# EUROPEAN PATENT APPLICATION

(11) **EP 2 457 998 A1**
(43) Date of publication of application: **30.05.2012**
(21) Application number: 10801854.0
(22) Date of filing: 23.07.2010
(51) Int. Cl.: C12N 5/07

(54) **METHODS FOR OBTAINING HEPATIC CELLS, HEPATIC ENDODERM CELLS AND HEPATIC PRECURSOR CELLS BY INDUCING THE DIFFERENTIATION**

(30) Priority: 23.07.2009 CN 200910089765; 24.07.2009 CN 200910089693; 24.07.2009 CN 200910089695
(71) Applicant: Beijing Huayuanbochuang Technology Co., Ltd., Beijing 100871 (CN)
(72) Inventor: DENG, Hongkui, Beijing 100871 (CN); DING, Mingxiao, Beijing 100871 (CN); ZHAO, Dongxin, Beijing 100871 (CN); CHEN, Song, Beijing 100871 (CN); SONG, Zhihua, Beijing 100871 (CN)
(74) Representative: HOFFMANN EITLE
(86) International application number: PCT/CN2010/001118
(87) International publication number: WO 2011/009294

(57) **Abstract**

The present invention discloses a method for inducing the differentiation of embryonic stem cells (ESC) or induced pluripotent stem cells (iPS cells) into hepatocytes, a method for inducing the differentiation of embryonic stem cells or induced pluripotent stem cells into hepatic endoderm cells, and a method for inducing the differentiation of embryonic stem cells (ESC) or induced pluripotent stem cells into hepatic progenitor cells. The present invention also provides the hepatocytes, hepatic endoderm cells and hepatic progenitor cells obtained by above methods, and the uses of these cells.

## Description

### Field of the Art

The invention relates to a method for inducing the differentiation of embryonic stem cells (ESC) or induced pluripotent stem cells (iPS cells) into hepatocytes, a method for inducing the differentiation of embryonic stem cells (ESC) or induced pluripotent stem cells (iPS cells) into hepatic endoderm cells, and a method for inducing the differentiation of embryonic stem cells (ESC) or induced pluripotent stem cells (iPS cells) into hepatic progenitor cells. The invention also involves the hepatocytes, hepatic endoderm cells and hepatic progenitor cells obtained by the above methods, and the use of these cells.

### Background of the invention

### Induced pluripotent stem cells

Induced pluripotent stem cells (iPS cells) and embryonic stem cells have very similar features, and possess a potential to differentiate into various cells *in vitro.* These types of cells can maintain the size of their cell populations or proliferate by cell division, and further differentiate into various specific cell types as well. The first mammal iPS cell strain was established in August, 2006. It was reported by Prof. Yamanaka's laboratory in Japan that somatic cells of mice can be converted to " induced pluripotent stem cells (iPS cells)" by transduction of four genes (Oct4, Sox2, Elf4 and c-Myc) (Takahashi, K. Cell 2006; 126, 663-676.). It has been demonstrated that these induced pluripotent stem cells (iPS cells) not only can be integrated into blastocysts and participate in the normal embryonic development and the formation of tissues and organs, but also can form chimeric mice under some specific conditions, In 2007, Thomson's laboratory and Yamanaka's laboratory almost simultaneously reported the establishment of human iPS cell line (Yu J, et al. Science 2007; 318:1917-1920, Takahashi K. Cell 2007; 131:861-872.). This type of cells has similar features with those of embryonic stem cells, and can differentiate into endoderm, mesoderm and ectoderm under specific induction conditions and generate teratoma. Up to now, the human iPS cell line has been established by researcher in many countries and regions.

Since iPS cells can proliferate unlimitedly in *vitro,* and can maintain the potential for multi-directional differentiation, adequate number of cells can be obtained by directed differentiation of iPS cells, so as to be used for cell transplantation therapy and gene therapy. If treatment can be carried out by obtaining somatic cells from patients, establishing the iPS cell line that shares the same genetic background as the patients, differentiating the iPS cells into the cell type that is desired by the patients and finally transplanting the desired cells back into the patients, then immunological rejection caused by exogenous transplantation can be avoided. Accomplishment of such a therapeutical cloning method will provide a new therapeutical pathway for many currently uncurable diseases, such as diabetes, leukemia, and cardiovascular diseases, etc. In addition, the investigation of human iPS cells will help providing an experimental platform, which is much better than animal models, for drug screening, pharmacological analysis, and toxicity evaluation, etc. It is demonstrated by the investigation that human iPS cells can differentiate into various cell types *in vitro,* such as nerve cells (Dimes JT. Science 2008; 321:1218-1221; Chambers SM. Nat Biotechnol 2009; 27:275-280; Karumbayaram S. Stem Cells 2009; 27:806-811; Hirami Y Neurosci Lett 2009; 458:126-131.), osteoblasts (Kamer E. J Cell Physiol 2009; 218:323-333.), myocardial cells (Zhang J. Circ Res 2009; 104:e30-41.), adipocytes (Taura D. FEBS Lett 2009; 583:1029-1033.), pancreatic cells (Tateishi K. J Biol Chem 2008; 283:31601-31607; Zhang D. Cell Res 2009; 19:429-438.), and hematopoietic cells (Taura D. Arterioscler Thromb Vasc Biol 2009; Choi KD. Stem Cells 2009;27:559-567.).

Differentiation of iPS cells into specific tissue cells is the key point for achieving the therapeutical cloning. Till now, plenty of experience has been accumulated for the differentiation of embryonic stem cells and iPS cells, and the differentiation of embryonic stem cells into hepatocytes has also made some progress, for example, the cells expressing the proteins specific to hepatocytes are obtained, which possess the functions of synthesizing glycogen, secreting albumin, and the like (Cai J. Hepatology 2007; 45:1229-1239.).

### Human embryonic stem cells and hepatic progenitor cells

Human embryonic stem cells, which can differentiate into all types of cells in human bodies, have the ability of unlimited proliferation and totipotence of differentiation. As a result, human embryonic stem cells have the potential for providing sources for all kinds of cells, which results in a remarkable application potential. For example, human embryonic stem cells can be used for studying the mechanism of cell lineage determination during development, or in the cell transplantation for all kinds of degenerative diseases. In the cell lineages differentiated from human embryonic stem cells, hepatocytes draw a special attention. This is because liver plays an important role in metabolism in human body, and possesses many critical functions, including glycogen synthesis, erythrocyte lysis, plasma protein synthesis, and detoxification, etc. Recently, numbers of research groups successfully accomplished the differentiation of human or mouse embryonic stem cells into hepatocyte lineage.

During the early stage of hepatic tissue development, hepatic progenitor cells are the major component of the hepatic parenchyma. Through a developmental study for mice and human, it is found that these hepatic progenitor cells are the common progenitors of mature hepatic parenchymal cells and epithelial cells of bile ducts in livers. The differentiation of hepatic progenitor cells into the two lineages, liver and bile duct, is determined gradually in the midterm of pregnancy. The features of hepatic progenitor cells have been preliminarily studied by isolating hepatic progenitor cells from embryonic livers of human and mice and culturing these cells *in vitro.* In the culture process *in vitro,* human hepatic progenitor cells show a strong potent ability of proliferation, and exhibit a stable phenotype. When placed under a suitable condition, hepatic progenitor cells can differentiate into hepatic parenchyma-like cells that express ALB and store glycogen; as well as into bile duct cells that express KRT19.

Although it haves been confirmed that hepatic progenitor cells have an proliferation ability and a dual-directional differentiation potential towards liver and bile duct, the origin and function of these hepatic progenitor cells are still questionable. This is perhaps mainly because hepatic progenitor cells can be obtained only by direct isolation from liver for now, and the shortness of early-stage human embryos dramatically limits the investigation in this field.

### Summary of the Invention

Based on the above state of prior art, the present invention provides:
1. A method for inducing the differentiation of embryonic stem cells (ESC) or induced pluripotent stem cells (iPS cells) into hepatocytes, comprising the following steps:
   1) culturing said embryonic stem cells (ESC) or induced pluripotent stem cells (iPS cells) in a basic cell culture medium containing activin A;
   2) transferring the cells obtained in step 1) into a basic cell culture medium containing insulin-transferrm-selenium salt (preferably sodium selenite) and activin A for cultivation;
   3) culturing the cells obtained in step 2) in a hepatocyte culture medium (HCM) containing fibroblast growth factor (FGF) and bone morphogenetic protein (BMP), so as to generate hepatic progenitor cells; and
   4) promoting the maturation of said hepatic progenitor cells obtained in step 3), so as to generate hepatocytes,
      wherein preferably said embryonic stem cells (ESC) or induced pluripotent stem cells (iPS) are mammal cells, more preferably mouse or human cells, and most preferably human cells, wherein in the case that said cells are human cells, preferably said activin A is human activin A, said fibroblast growth factor is human fibroblast growth factor, and said bone morphogenetic protein is human bone morphogenetic protein.
2. The method according to the above Item 1, wherein said basic cell culture medium is selected from the group consisting of MEM, DMEM, BME, DMEM / F12, RPMI1640 and Fischer's.
3. The method according to the above Item 1 or 2, wherein the concentration of said activin A in said basic cell culture medium is 10∼500 ng/ml.
4. The method according to the above Item 3, wherein said insulin-transferrin-selenium salt is added as a mixed supplementary liquid, and the volume ratio of said insulin-transferrin-selenium salt to said basic cell culture medium is 0.01∼20%.
5. The method according to the above Item 4, wherein said fibroblast growth factor is acidic fibroblast growth factor, fibroblast growth factor 2 or fibroblast growth factor 4; and said bone morphogenetic protein is bone morphogenetic protein 2 or bone morphogenetic protein 4.
6. The method according to the above Item 5, wherein the amount of said fibroblast growth factor (FGF) is 5∼100 ng/ml said hepatocyte culture medium; and the amount of bone morphogenetic protein (BMP) is 5∼100 ng/ml said hepatocyte culture medium.
7. The method according to the above Item 6, wherein promotion of maturation of said hepatocytes is carried out by culturing said hepatocytes in hepatocyte culture medium containing hepatocyte growth factor (preferably human hepatocyte growth factor) and keratinocyte growth factor (preferably human keratinocyte growth factor) so as to obtain proliferated hepatic progenitor cells; transferring the hepatic progenitor cells into a hepatocyte culture medium containing oncostatin M and dexamethasone for cultivation, then transferring the cells into differentiation medium V and obtaining mature hepatocytes; wherein said differentiation medium V is a basic culture medium containing (0.1-10)ml/100ml N2, (0.1-20)ml/100ml B27, 0.5-2mM glutamine, (0.1-10)ml/100ml nonessential amino acid, 0.05-0.2 mM β-mercaptoethanol, 1-100ng/ml oncostatin M(OSM) and 0.05-1µM dexamethasone (Dex), pH 7.2-7.6.
8. The method according to the above Item 7, wherein the amount of said hepatocyte growth factor is 5∼100 ng/ml said hepatocyte culture medium; the amount of said keratinocyte growth factor is 5∼100 ng/ml said hepatocyte culture medium, the amount of said oncostatin M is 1∼100 ng/ml said hepatocyte culture medium; the concentration of said dexamethasone in said hepatocyte culture medium is 0.05∼1 µM.
9. Hepatocytes obtained by the method according to any of the above Items 1-8,
   wherein preferably said hepatocytes express marker molecules ASP, Alb, CK8, CK18, CK19, HNF4α, and/or GAPDH of hepatocytes, more preferably said hepatocytes have glycogen synthesis and storage function, urea synthesis function, leukocyte secretion function and/or P450 enzyme activity in response to drug induction.
10. Use of the hepatocytes obtained by the method according to any of the above Items 1-8 in preparation of artificial livers, test of drug toxicity or drug screening.
11. A method for inducing the differentiation of embryonic stem cells (ESC) or induced pluripotent stem cells (iPS cells) into hepatic endoderm cells, comprising the following steps:
   1) culturing said embryonic stem cells (ESC) or induced pluripotent stem cells (iPS cells) in a basic cell culture medium containing activin A;
   2) transferring the cells obtained in step 1) into a basic cell culture medium containing insulin-transferrin-selenium salt (preferably sodium selenite) and activin A for cultivation; and
   3) culturing the cells obtained in step 2) in a hepatic endoderm cell inducing medium containing fibroblast growth factor (FGF) and bone morphogenetic protein (BMP), so as to generate hepatic endoderm cells,
      wherein preferably said embryonic stem cells (ESC) or induced pluripotent stem cells (iPS cells) are mammal cells, more preferably mouse or human cells, and most preferably human cells, wherein in the case that said cells are human cells, preferably said activin A is human activin A, said fibroblast growth factor is human fibroblast growth factor, and said bone morphogenetic protein is human bone morphogenetic protein.
12. The method according to the above Item 11, wherein said basic cell culture medium used in steps 1) and 2) further contains bovine serum albumin component V, wherein preferably said fibroblast growth factor is acidic fibroblast growth factor, fibroblast growth factor 2 or fibroblast growth factor 4; and said bone morphogenetic protein is bone morphogenetic protein 2 or bone morphogenetic protein 4.
13. The method according to the above Item 11 or 12, wherein in step 2), the cells obtained in step 1) are first transferred into a basic cell culture medium containing insulin-transferrin-selenium salt at a first concentration and activin A so as to culture the cells, then the resultant cells are cultured in a basic cell culture medium containing insulin-transferrin-selenium salt at a second concentration and activin A, said second concentration is higher than said first concentration.
14. The method according to the above Item 13, wherein the medium used in step 1) is a basic cell culture medium containing 0.02%-1% w/w of bovine serum albumin component V and 50-200 ng/ml human activin A; the medium used in step 2) is a basic cell culture medium containing 0.02%-1% w/w of bovine serum albumin component V, 0.05%-0.5% v/v of insulin-transferrin-sodium selenite mixed supplementary liquid and 50-200ng/ml human activin A, and a basic cell culture medium containing 0.02%-1% w/w of bovine serum albumin component V, 0.5%-2% v/v of insulin-transferrin-sodium selenite mined supplementary liquid and 50-200ng/ml human activin A, respectively; said hepatic endoderm cell inducing medium is hepatocyte culture medium containing 20-60ng/ml human fibroblast growth factor -4 and 10-30ng/ml human bone morphogenetic protein -2.
15. The method according to any of the above Items 11-14, wherein said basic cell culture medium is selected from the group consisting of MEM, DMEM, BME, DMEM / F12, RPMI1640 and Fischer's.
16. The method according to the above Item 11, further comprising a step of sorting the cells expressing the surface protein N-cadherin by using a flow cytometer after step 3).
17. The method according to the above Item 11., wherein the cells are cultured for 24 hours, 48 hours and 5 days in steps 1), 2) and 3), respectively.
18. The method according to the above Item 11, wherein said embryonic stem cell (ESC) is human embryonic stem cell, said human embryonic stem cell is commercially available human embryonic stem cell line; preferably is one of the following cell lines: BG01, BG02, BG03, BG04, SA01, SA02, SA03, ES01, ES02, ES03, ES04, ES05, ES06, TE03, TE32, TE33, TE04, TE06, TE62, TE07, TE72, UC01, UC06, WA01,WA07, WA09, WA13 and WA14; wherein the accession numbers are NIH numbers.
19. Hepatic endoderm cells obtained by the differentiation of human embryonic stem cells or human induced pluripotent stem cells by the method according to any of the above Items 11-18, wherein preferably the hepatic endoderm cells express at least 3 types of marker protein of hepatic endoderm cells, i.e. α-fetoprotein, hepatocyte nuclearfactor 4A and N-cadherin.
20. The hepatic endoderm cells according to the above Item 19, wherein said hepatic endoderm cells express α-fetoprotein, albumin, hepatocyte nuclearfactor 4A, hepatocyte nuclearfactor 3B and N-cadherin.
21. Use of the hepatic endoderm cells according to the above Item 19 or 20 in preparation of hepatic parenchyma-like cells or bile duct cells.
22. A method for inducing the differentiation of embryonic stem cells (ESC) or induced pluripotent stem cells (iPS cells) into hepatic progenitor cells, comprising:
   1) culturing said embryonic stem cells (ESC) or induced pluripotent stem cells (iPS cells) in a basic cell culture medium containing activin A;
   2) transferring the cells obtained in step 1) into a basic cell culture medium containing insulin-transferrm-seienium salt (preferably sodium selenite) and activin A for cultivation;
   3) culturing the cells obtained in step 2) in a hepatic endoderm cell inducing medium containing fibroblast growth factor (FGF) and bone morphogenetic protein (BMP), so as to generate hepatic endoderm cells; and
   4) culture the hepatic endoderm cells obtained in step 3) with a hepatic progenitor cell culture medium on STO cell feeder layer,
      wherein preferably said embryonic stem cells (ESC) or induced pluripotent stem cells (iPS cells) are mammal cells, more preferably mouse or human cells, and most preferably human cells, wherein in the case that said cells are human cells, preferably said activin A is human activin A, said fibroblast growth factor is human fibroblast growth factor, and said bone morphogenetic protein is human bone morphogenetic protein.
23. The method according to the above Item 22, wherein said basic cell culture medium used in steps 1) and 2) further contains bovine serum albumin component V, wherein preferably said fibroblast growth factor is acidic fibroblast growth factor, fibroblast growth factor 2 or fibroblast growth factor 4; said bone morphogenetic protein is bone morphogenetic protein 2 or bone morphogenetic protein 4.
24. The method according to the above Item 22 or 23, wherein in step 2), the cells obtained in step 1) are first transferred into a basic cell culture medium containing insulin-transferrin-selenium salt at a first concentration and activin A so as to culture the cells, then the resultant cells are cultured in a basic cell culture medium containing insulin-transferrin-selenium salt at a second concentration and activin A, said second concentration is higher than said first concentration.
25. The method according to the above Item 24, wherein the medium used in step 1) is a basic cell culture medium containing 0.02%-1% w/w of bovine serum albumin component V and 50-200 ng/ml human activin A; the medium used in step 2) is a basic cell culture medium containing 0.02%-1% w/w of bovine serum albumin component V, 0.05%-0.5% v/v of insulin-transferrin-sodium selenite mixed supplementary liquid and 50-200ng/ml human activin A, and a basic cell culture medium containing 0.02%-1% w/w of bovine serum albumin component V, 0.5%-2% v/v of insulin-transferrin-sodium selenite mixed supplementary liquid and 50-200ng/ml human activin A, respectively; said hepatic endoderm cell inducing medium is a hepatocyte culture medium containing 20-60ng/ml human fibroblast growth factor -4 and 10-30ng/ml human bone morphogenetic protein -2, said hepatic progenitor cell culture medium is a basic cell culture medium containing 5-25 mM HEPES, 0,5%-2% v/v of insulin-transferrin-sodium selenite mixed supplementary liquid, 0.02%-1% w/w of bovine serum albumin component V, 2-20 mM nicotinamide, 0.2-2mM diphosphorylated ascorbic acid, 0.02-0.2µM dexamethasone, and 5-40ng/ml EGF.
26. The method according to any of the above Items 22-25, wherein said basic cell culture medium is selected from the group consisting of MEM, DEEM, BME, DMEM / F12, RPMI1640 and Fischer's.
27. The method according to the above Item 22, further comprising a step of sorting the cells expressing the surface protein N-cadherin by using a flow cytometer after step 3).
28. The method according to the above Item 22, wherein the cells are cultured for 24 hours, 48 hours and 5 days in steps 1), 2) and 3), respectively.
29. The method according to any of the above Items 22-28, further comprising a passage step of the hepatic progenitor cells; the method for the passage of the hepatic progenitor cells comprises the steps of digesting said hepatic progenitor cells with trypsin-EDTA solution, and culturing the resultant cells on a hepatic progenitor cell culture medium with STO cells used as the feeder layer.
30. The method according to the above Item 22, wherein said embryonic stem cell (ESC) is human embryonic stem cell, said human embryonic stem cell is commercially available human embryonic stem cell line; preferably is one of the following cell lines: BG01, BG02, BG03, BG04, SA01, SA02, SA03, ES01, ES02, ES03, ES04, ES05, ES06, TE03, TE32, TE33, TE04, TE06, TE62, TE07, TB72, UC01, UC06, WA01,WA07, WA09, WA13 and WA14; wherein the accession numbers are NIH numbers.
31. Hepatic progenitor cells obtained by the differentiation of human embryonic stem cells or human induced pluripotent stem cells by the method according to any of the above Items 22-30, wherein preferably the hepatic progenitor cells are hepatic progenitor cells expressing α-fetoprotein, keratin 19 and keratin 7, and possess a proliferation ability and a dual-directional differentiation potential towards hepatic parenchyma-like cells and bile duct-like cells.
32. Use of the hepatic progenitor cells according to the above Item 31 in preparation of hepatic parenchyma-like cells or bile duct cells.

### Detailed Description of the Invention

One objective of the invention is to provide a method for inducing the differentiation of induced pluripotent stem cells into hepatocytes, and a potential of the hepatocytes obtained by this method for screening drugs.

The method for inducing the differentiation of induced pluripotent stem cells into hepatocytes provided by the invention comprises the following steps: induced pluripotent stem cells are cultured in differentiation medium I, then transferred into differentiation medium I containing insulm-transferrin-selemum salt, further cultured in a hepatocyte culture medium (HCM) containing fibroblast growth factor and bone morphogenetic protein, so as to generate hepatic progenitor cells; said hepatic progenitor cells are promoted to become mature, so as to generate hepatocytes; wherein the differentiation medium I is a basic cell culture medium containing activin A.

Among others, said basic cell culture medium is MEM(Minimum Essential Medium), DMEM, BME(Basal Medium Eagle), DMEM/F12, RPMI1640 or Fischer's (Fischer's Medium), which is well known in the prior art and commercially available from companies such as Sigma Aldrich, Invitrogen, Gibco, etc.

The amount of said activin A can be 10-500ng/ml said differentiation medium I; the volume ration of said insulin-transferrin-selenium salt (preferably sodium selenite) to said differentiation medium I is 0.01-20%.

Said fibroblast growth factor (FGF) is acidic fibroblast growth factor, fibroblast growth factor 2 or fibroblast growth factor 4; and said bone morphogenetic protein (BMP) is bone morphogenetic protein 2 or bone morphogenetic protein 4.

The amount of said fibroblast growth factor (FGF) can be 5-100ng/ml said hepatocyte culture medium; the amount of said bone morphogenetic protein (BMP) can be 5-100ng/ml said hepatocyte culture medium.

Maturation of the hepatic progenitor cells can be promoted by existing methods. Alternatively, the hepatic progenitor cells can be cultured in a hepatocyte culture medium containing hepatocyte growth factor (HGF) and keratinocyte growth factor (KGF) so as to obtain proliferated hepatic progenitor cells; the proliferated hepatic progenitor cells can be transferred into a hepatocyte culture medium containing oncostatin M and dexamethasone, and then transferred into differentiation medium V so as to generate mature hepatocytes. The differentiation medium V is a hepatocyte culture medium or a basic culture medium containing 0.1-10%(volume percentage) N2(purchased from Invitrogen, catalog No: 17502-048), 0.1-20%(volume percentage) B27(purchased from Invitrogen, catalog No: 17504-044), 0.5-2mM glutamine, 0.1-10%(volume percentage) nonessential amino acid, 0.05-0.2mM β- mercaptoethanol, 1-100ng/ml oncostatin M (OSM) and 0.05-1µM dexamethasone (Dex).

The hepatocyte growth factor (HGF) can be present in an amount of 5-100ng/ml said hepatocyte culture medium; keratmocyte growth factor (HGF) can be present in an amount of 5-100ng/ml said hepatocyte culture medium; oncostatin M can be present in an amount of 1-100ng/ml said hepatocyte culture medium or basic culture medium; dexamethasone (Dex) can be present in the hepatocyte culture medium or basic culture medium at a concentration of 0.05-1µM.

The hepatocytes, obtained by the above methods, that express normal hepatocyte marker molecules such as AFP(α-fetoprotein), Alb(albumin (ALB)), CK18(cytokeratin (keratin)18), CK8(cytokeratin (keratin)8), CK19(cytokeratizi (keratin)19), AAT(α-antitrypsin), CYP3A4 (liver drug enzyme), hepatocyte nuclear factor 4A (HNF4A, or HNF4α), GAPDH (glyceraldehyde-3-phosphate dehydrogenase), etc. or have the normal hepatocyte functions such as glycogen synthesis or storage, urea synthesis, albumin secretion, etc. also fall into the protection scope of the invention.

Therefore, the invention, in the first aspect, provides:
1. A method for inducing the differentiation of induced pluripotent stem cells into hepatocytes, comprising the following steps: the induced pluripotent stem cells (iPS cells) are cultured in differentiation medium I, transferred into differentiation medium I containing insulin-transferrin-selenium salt, afterwards cultured in a hepatocyte culture medium containing fibroblast growth factor and bone morphogenetic protein so as to generate hepatic progenitor cells, the resultant hepatic progenitor cells are then promoted to become mature so as to obtain hepatocytes; the differentiation medium I is a basic cell culture medium containing activin A.
2. The method according to the above Item 1, **characterized in that**: the basic cell culture medium is MEM, DMEM, BME, DMEM / F12, RPMI1640 or Fischer's.
3. The method according to the above Item 1 or 2, **characterized in that**: activin A is in an amount of 10-500ng/ml said differentiation medium I.
4. The method according to the above Item 3, **characterized in that**: the volume ratio of said insulin-transferrin-selenium salt to said Differentiation medium I is (0.01-20)%.
5. The method according to the above Item 4, **characterized in that**: said fibroblast growth factor is acidic fibroblast growth factor, fibroblast growth factor 2 or fibroblast growth factor 4; said bone morphogenetic protein is bone morphogenetic protein 2 or bone morphogenetic protein 4.
6. The method according to the above Item 5, **characterized in that**: said fibroblast growth factor (FGF) can be in an amount of 5-100ng/ml said hepatocyte culture medium; said bone morphogenetic protein (BMP) can be in an amount of 5-100ng/ml said hepatocyte culture medium.
7. The method according to the above Item 6, **characterized in that**: promotion of maturation of said hepatocytes is carried out by culturing said hepatocytes in a hepatocyte culture medium containing hepatocyte growth factor and keratinocyte growth factor so as to obtain proliferated hepatic progenitor cells; transferring the hepatic progenitor cells into a hepatocyte culture medium containing oncostatin M and dexamethasone for cultivation, then transferring the cells into differentiation medium V and obtaining mature hepatocytes; wherein said differentiation medium V is a basic culture medium containing (0.1-10)ml/100ml N2, (0.1-20)ml/100ml B27, 0.5-2mM glutamine, (0.1-10)ml/100ml nonessential amino acid, 0.05-0.2 mM β-mercaptoethanol, 1-100ng/ml oncostatin M (OSM) and 0.05-1µM dexamethasone (Dex), pH 7.2-7.6.
8. The method according to the above Item 7, **characterized in that**: the amount of said hepatocyte growth factor is 5∼100 ng/ml said hepatocyte culture medium; the amount of said keratinocyte growth factor is 5∼100 ng/ml said hepatocyte culture medium, the amount of said oncostatin M is 1∼100 ng/ml said hepatocyte culture medium; the amount of said dexamethasone in said hepatocyte culture medium is 0.05∼1µM.
9. Hepatocytes obtained by the method of any of the above Items 1∼8.
10. Use of the method of any of the above Items 1∼8 in preparation of artificial livers or drug screening.

In the inventive method for inducing the differentiation of induced pluripotent stem cells into hepatocytes, iPS cells are induced by activin A to efficiently differentiate into definitive endoderm cells, and further differentiate into early-stage hepatocytes expressing albumin under the cooperation of fibroblast growth factor and bone morphogenetic protein. The differentiated early-stage hepatocytes can continue to proliferate with the promotion by hepatocyte growth factor and keratinocyte growth factor, and further maturated with the co-promotion by OSM, Dex and N2, B27. The obtained differentiated cells is of the typical morphology of hepatocytes, and more than about 60% of these cells express marker proteins CK8(cytokeratin (keratin)8), A1b, C18 and AFP of the early-stage hepatocytes. The hepatocytes that are differentiated from iPS cells also express marker molecules AAT and CYP3A4 of mature hepatocytes. The entire differentiation process is very similar to the early stage of liver development. The hepatocytes obtained by the present method have an inducible CYP450 enzyme activity, which could make a response to the induction of drugs. The inventive method for inducing the differentiation of induced pluripotent stem cells (iPS cells) into hepatocytes has the advantages of short period, high differentiation efficiency, safety and stableness. The hepatocytes that are obtained by differentiation can be used in the treatment of liver diseases by cell transplantation, artificial livers, and toxicity test of drugs, etc. Additionally, the entire differentiation process can be used for investigating the early stage of human embryonic liver development, which has a wide application prospect.

Another objective of the invention is to provide a hepatic endoderm cell and the preparation and purification methods thereof.

The hepatic endoderm cell provided by the invention is the hepatic endoderm cell obtained by the differentiation of human embryonic stem cells (human ESCs) or human induced pluripotent stem cells (human iPS cells), which expresses at least three types of marker protein, i.e. α-fetoprotein (AFP), hepatocyte nuclearfactor 4A (HNF4A) and N-cadherin.

The hepatic endoderm cells can express albumin (ALB) and hepatocyte nuclear factor 3B (FOXA2) as well.

In particular the human embryonic stem cells are commercially available human embryonic stem cell lines, as shown in Table 1.

**Table 1. Commercially available human embryonic stem cell lines**

| Provider | Accession number | NIH number |
|---|---|---|
| BresaGen, Inc. | hESBGN-01, | BG01, |
| | hESBGN-02, | BG02, |
| | hESBGN-03, | BG03, |
| | bESBGN-04 | BG04 |
| Cellartis AB | Sahlgrenska 1, | SA01, |
| | Sahlgrenska 2, | SA02, |
| | Sahlgrenska 3 | SA03 |
| ES Cell | HES-1, | ES01, |
| International | HES-2, | ES02, |
| | HES-3, | ES03, |
| | HES-4, | ES04, |
| | HES-5, | ES05, |
| | HES-6 | ES06 |
| Technion-Israel | I3, | TE03, |
| Institute of | I3.2, | TE32, |
| Technology | I3.3, | TE33, |
| | I4, | TE04, |
| | I6, | TE06, |
| | I6.2, | TE62, |
| | J3, | TE07, |
| | J3.2 | TE72 |
| University of | HGF-1, | UC01, |
| California, San | HSF-6 | UC06 |
| Francisco | | |
| Wisconsin Alumni | H1, | WA01, |
| Research | H7, | WA07, |
| Foundation (WiCell | H9, | WA09, |
| Research Institute) | H13, | We13, |
| | H14 | WA14 |

Another objective of the invention is to provide a hepatic endoderm cell and the preparation and purification methods thereof.

The method for preparing and purifying the hepatic endoderm cells of the invention comprises the steps of:
1) culturing human embryonic stem cells or induced pluripotent stem cells on endoderm inducing medium I;
2) culturing the cells obtained in step 1) on endoderm inducing medium II;
3) culturing the cells obtained in step 2) on endoderm inducing medium III;
4) culturing the cells obtained in step 3) on hepatic endoderm cell inducing medium;
   the endoderm inducing medium I is a basic cell culture medium containing 0.02%-1% w/w of bovine serum albumin component V and 50-200ng/ml human activin A; wherein, the amount of the bovine serum albumin component V is preferably 0.02%-0.1% w/w, particularly preferably 0.05% w/w; and the amount of human activin A is preferably 80-150ng/ml, particularly preferably 100ng/ml;
   the endoderm inducing medium II is a basic cell culture medium containing 0.02%-1% w/w of bovine serum albumin component V, 0.05%-0.5% v/v of insulin-transferrin-sodium selenite mixed supplementary liquid and 50-200ng/ml human activin A; wherein the amount of the bovine serum albumin component V is preferably 0.02%-0.1 %, particularly preferably 0.05%; the amount of the human activirl A is preferably 80-150ng/ml, particularly preferably 100ng/ml; and the amount of the insulin-transferrin-sodium selenite mixed supplementary liquid is preferably 0.05%-0.15%, particularly preferably 0.1%;
   the endoderm inducing medium III is a basic cell culture medium containing 0.02%-1 % w/w of bovine serum albumin component V, 0.5%-2% v/v of insulin-transferrin-sodium selenite mixed supplementary liquid and 50-200ng/ml human activin A; wherein, the amount of the bovine serum albumin component V is preferably 0.02%-0.1%, particularly preferably 0,05%; the amount of the human activin A is preferably 80-150ng/ml, particularly preferably 100ng/ml; the amount of the insulin-transferrin-sodium selenite mixed supplementary liquid is preferably 0.8%-1.5%, particularly preferably 1%;
   the hepatic endoderm cell inducing medium is the hepatocyte culture medium containing 20-60ng/ml human fibroblast growth factor -4 and 10-30ng/ml human bone morphogenetic protein -2; wherein, the amount of the human fibroblast growth factor -4 is preferably 30ng/ml, the amount of the bone morphogenetic protein -2 is preferably 20ng/ml.

The above endoderm inducing medium I, endoderm inducing medium II, endoderm inducing medium III and hepatic endoderm cell inducing medium can have a pH conventionally used for culturing mammal cells, for example pH 7.2-7.6.

Among others, the method also comprises a step of sorting the cells expressing the surface protein N-cadherin by using a flow cytometer.

The obtained hepatic endoderm cells are digested with trypsin (to which no EDTA but 2mM calcium ion has been added), then the cells expressing the surface protein N-cadherin are sorted by using a flow cytometer.

The human embryonic stem cells are shown in Table 1. The basic cell culture medium can be MEM, DMEM, BME, DMEM / FI2, RPMI1640 or Fischer's medium.

In said method, the human embryonic stem cells or induced pluripotent stem cells are cultured on endoderm inducing medium I for 24h. In said method, the cells obtained in step 1) are cultured on endoderm inducing medium II for 24h. In said method, the cells obtained in step 2) are cultured on endoderm inducing medium III for 24h. In said method, the cells obtained in step 3) are cultured on hepatic endoderm cell inducing medium for 5 days.

The culture media for the preparation of hepatic endoderm cells from human embryonic stem cells or induced pluripotent stem cells also fall into the protection scope of the invention. The culture media for the preparation of human embryonic stem cells or induced pluripotent stem cells from hepatic endoderm cells consists of the above endoderm inducing medium I, the above endoderm inducing medium II, the above endoderm inducing medium III and the above hepatic endoderm cell inducing medium.

In the present invention, the differentiation process of human embryonic stem cells into hepatic lineage is detected, and the generation of hepatic endoderm cells during this differentiation process is identified. One surface marker protein, N-cadherin, is found, which can effectively represent the hepatic endoderm cells that are firstly generated and are AFP-positive in the differentiation process. Accordingly, hepatic endoderm cells can be separated and purified from miscellaneous human embryonic stem cells by using a flow cell sorting method.

Therefore, the invention, in the second aspect, provides:
1. Hepatic endoderm cells, which are obtained by the differentiation of human embryonic stem cells or human induced pluripotent stem cells, and express at least three types of marker protein of the hepatic endoderm cells, i.e. α-fetoprotein, hepatocyte neclear factor 4A and N-cadherin.
2. The hepatic endoderm cells according to the above Item 1, **characterized in that**: said hepatic endoderm cells express α-fetoprotein, albumin, hepatocyte neclear factor 4A, hepatocyte nuclear factor 3B and N-cadherin.
3. The hepatic endoderm cells according to the above Item 1 or 2, **characterized in that**: said human embryonic stem cell is a human embryonic stem cell line.
4. The hepatic endoderm cells according to the above Item 3, **characterized in that**: said human embryonic stem cell line is any cell line of BG01, BG02, BG03, BG04, SA01, SA02, SA03, ES01, ES02, ES03, ES04, ES05, ES06, TE03, TE32, TE33, TE04, TE06, TE62, TE07, TE72, UC01, UC06, WA01, WA07, WA09, WA13 and WA14; wherein the accession numbers are NIH numbers.
5. A method for preparing the hepatic endoderm cells according to any of the above Items 1 to 4, comprises the steps of:
   1) culturing human embryonic stem cells or induced pluripotent stem cells on endoderm inducing medium I;
   2) culturing the cells obtained in step 1) on endoderm inducing medium II;
   3) culturing the cells obtained in step 2) on endoderm inducing medium III;
   4) culturing the cells obtained in step 3) on hepatic endoderm cell inducing medium, so as to generate hepatic endoderm cells;
      the endoderm inducing medium I is a basic cell culture medium containing 0.02%-1% w/w of bovine serum albumin component V and 50-200ng/ml human activin A; the endoderm inducing medium II is a basic cell culture medium containing 0.02%-1% w/w of bovine serum albumin component V, 0.05%-0.5% v/v of insulin-transferrin-sodium selenite mixed supplementary liquid and 50-200ng/ml human activin A; the endoderm inducing medium III is a basic cell culture medium containing 0.02%-1% w/w of bovine serum albumin component V, 0.5%-2% v/v of insulin-transferrin-sodium selenite fixed supplementary liquid and 50-200ng/ml human activin A; and the hepatic endoderm cell inducing medium is a hepatocyte culture medium containing 20-60ng/ml human fibroblast growth factor -4 and 10-30ng/ml human bone morphogenetic protein -2.
6. The method according to above Item 5, **characterized in that**: the method further comprises a step of sorting the cells expressing the surface protein N-cadherin by using a flow cytometer.
7. The method according to the above Item 5 or 6, **characterized in that**: said basic cell culture medium is MEM, DMEM, BME, DMEM / F12, RPMI1640 or Fischer's.
8. The method according to any of the above Items 5-7, **characterized in that**: in said method, the human embryonic stem cells or induced pluripotent stem cells are cultured on endoderm inducing medium I for 24h; in said method, the cells obtained in step 1) are cultured on endoderm inducing medium II for 24h; in said method, the cells obtained in step 2) are cultured on endoderm inducing medium III for 24h; in said method, the cells obtained in step 3) are cultured on hepatic endoderm cell inducing medium for 5 days.
9. The method according to any of the above Items 5-8, **characterized in that**: said human embryonic stem cells can be commercially available human embryonic stem cell lines;
   preferably the commercially available human embryonic stem cell line is any cell line of BG01, BG02, BG03, BG04, SA01, SA02, SA03, ES01, ES02, ES03, ES04, ES05, ES06, TE03 , TE32, TE33, TE04, TE06, TE62, TE07, TE72, UC01, UC06, WA01, WA07, WA09, WA13 and WA14; the accession numbers are NIH numbers.
10. A culture medium for preparing hepatic endoderm cells from human embryonic stem cells or induced pluripotent stem cells, which consists of endoderm inducing medium I, endoderm inducing medium II, endoderm inducing medium III and hepatic endoderm cell inducing medium, wherein the endoderm inducing medium I is a basic cell culture medium containing 0.02%-1% w/w of bovine serum albumin component V and 50-200ng/ml human activin A; the endoderm inducing medium II is a basic cell culture medium containing 0.02%-1% w/w of bovine serum albumin component V, 0.05%-0.5% v/v of insulin-transrerrin-sodium selenite mixed supplementary liquid and 50-200ng/ml human activin A; the endoderm inducing medium III is a basic cell culture medium containing 0.02%-1% w/w of bovine serum albumin component V, 0.5%-2% v/v of insulin-transiemn-sodium selenite mixed supplementary liquid and 50-200ng/ml human activin A; and the hepatic endoderm cell inducing medium is a hepatocyte culture medium containing 20-60ng/ml human fibroblast growth factor -4 and 10-30ng/ml human bone morphogenetic protein -2.
11. Use of the cells according to any of the above Items 1-4, the method according to any of the above Items 5-9, or the culture medium according to above Item 10 in preparation of hepatocyte-like cells or bile duct cells.

The inventive hepatic endoderm cells are the hepatic endoderm cells obtained by differentiation of human embryonic stem cells or induced pluripotent stem cells, and express at least three types of marker protein i.e. α-fetoprotein, hepatocyte neclear factor 4A and N-cadherin. Hepatic progenitor cells can be obtained by further culturing the hepatic endoderm of the invention. These hepatic progenitor cells have the potential to differentiate into hepatic parenchyma or bile duct *in vitro.*

Yet another objective of the invention is to provide a hepatic progenitor cell and the preparation method and application thereof.

The inventive hepatic progenitor cells are cells that are obtained by Differentiation of human embryonic stem cells (human ESCs) or human induced pluripotent stem cells (human iPS cells) and express the early-stage hepatic marker protein i.e. α-fetoprotein (AFP) and the marker proteins of bile duct i.e. keratin 19(KRT19) and keratin 7(KRT7). These cells also have a proliferation ability and a dual-directional differentiation potential towards hepatic parenchyma-like cells and bile duct-like cells.

Another objective of the invention is to provide a method for preparing hepatic progenitor cells.

The inventive method for preparing hepatic progenitor cells comprises the steps of:
1) culturing human embryonic stem cells or induced pluripotent stem cells on endoderm inducing medium I;
2) culturing the cells obtained in step 1) on endoderm inducing medium II;
3) culturing the cells obtained in step 2) on endoderm inducing medium III;
4) culturing the cells obtained in step 3) on hepatic endoderm cell inducing medium, so as to generate hepatic endoderm cells;
5) culturing the hepatic endoderm cells with the hepatic progenitor cell culture medium on STO cells as a feeder layer, so as to generate hepatic progenitor cells.

The endoderm inducing medium I is a basic cell culture medium containing 0.02%-1% w/w of bovine serum albumin component V and 50-200ng/ml human activin A; wherein, the amount of the bovine serum albumin component V is preferably 0.02%-0.1% w/w, particularly preferably 0.05% w/w; and the amount of human activin A is preferably 80-150ng/ml, particularly preferably 100ng/ml;
the endoderm inducing medium II is a basic cell culture medium containing 0.02%-1% w/w of bovine serum albumin component V, 0.05%-0.5% v/v of insulin-transferrin-sodium selenite mixed supplementary liquid and 50-200ng/ml human activin A; wherein, the amount of the bovine serum albumin component V is preferably 0.02%-0.1%, particularly preferably 0.05%; the amount of the human activin A is preferably 80-150ng/ml, particularly preferably 100ng/ml; and the amount of the insulin-transferrin-sodium selenite mixed supplementary liquid is preferably 0.05%-0.15%, particularly preferably 0.1%;
the endoderm inducing medium III is a basic cell culture medium containing 0.02%-1% w/w of bovine serum albumin component V, 0.5%-2% v/v of insulin-transferrin-sodium selenite mixed supplementary liquid and 50-200ng/ml human activin A; wherein, the amount of the bovine serum albumin component V is preferably 0.02%-0.1%, particularly preferably 0.05%; the amount of the human activin A is preferably 80-150ng/ml, particularly preferably 100ng/ml; the amount of the insulin-transferrin-sodium selenite mixed supplementary liquid is preferably 0.8%-1.5%, particularly preferably 1%;
the hepatic endoderm cell inducing medium is a hepatocyte culture medium containing 20-60ng/ml human fibroblast growth factor -4 and 10-30ng/ml human bone morphogenetic protein -2; wherein, the amount of the human fibroblast growth factor -4 is preferably 30ng/ml, the amount of the bone morphogenetic protein -2 is preferably 20ng/ml;
the hepatic progenitor cell culture medium is a basic cell culture medium containing 5-25 mM HEPES, 0.5%-2% v/v of insulin-transferrin-sodium selenite mixed supplementary liquid, 0.02%-1% w/w of bovine serum albumin component V, 2-20 mM nicotinamide, 0.2-2mM diphosphorylated ascorbic acid, 0.02-0.2µM dexamethasone, and 5-40ng/ml EGF; wherein, the amount of HEPES is preferably 9-12 mM, particularly preferably 10 mM; the amount of msulin-transferrin-sodium selenite mixed supplementary liquid is preferably 0.8%-1.5%, particularly preferably 1%; the amount of bovine serum albumin component V is preferably 0.02%-0.1%, particularly preferably 0.05%; the amount of nicotinamide is preferably 8-14mM, particularly preferably 11mM; the amount of diphosphorylated ascorbic acid is preferably 0.8-1.5mM, particularly preferably 1mM; the amount of dexamethasone is preferably 0.08-0.15µM, particularly preferably 0.1µM; the amount of EGF is preferably 8-15 ng/ml, particularly preferably 10 ng/ml.

The above endoderm inducing medium I, endoderm inducing medium II, endoderm inducing medium III, hepatic endoderm cell inducing medium and hepatic progenitor cell culture medium can have a pH conventionally used for culturing mammal cells, for example pH 7.2-7.6.

Said method may further comprise a step of sorting the cells expressing the surface protein N-cadherin by using a flow cytometer between steps 3) and 4). The human embryonic stem cells are shown in Table 1. The basic cell culture medium can be MEM, DMEM, BME, DMEM / F12, RPMI1640 or Fischer's.

In said method, the human embryonic stem cells or induced pluripotent stem cells are cultured on endoderm inducing medium I for 24h. In said method, the cells obtained in step 1) are cultured on endoderm inducing medium II for 24h. In said method, the cells obtained in step 2) are cultured on endoderm inducing medium III for 24h. In said method, the cells obtained in step 3) are cultured on hepatic endoderm cell inducing medium for 5 days. In said method, the hepatic endoderm cells of step 4) are cultured with hepatic progenitor cell culture medium on STO cells as the feeder layer, so as to generate hepatic progenitor cells.

Said method may further comprise a passage method of hepatic progenitor cells. The passage method of hepatic progenitor cells comprises the following steps: the hepatic progenitor cells are digested with trypsin-EDTA solution (Invitrogen Co. U.S.A.), then cultured on the hepatic progenitor cell culture medium with STO cells as the feeder layer.

The media, consisting of the above endoderm inducing medium I, the above endoderm inducing medium II, the above endoderm inducing medium III, the above hepatic endoderm inducing medium and the above hepatic progenitor cell culture medium, are used for generating hepatic progenitor cells from human embryonic stem cells or induced pluripotent stem cells and fall into the protection scope of the invention.

In the present invention, the differentiation process of human embryonic stem cells into hepatic lineage is detected, and the generation of hepatic progenitor cells is identified during this differentiation process. One surface marker protein, N-cadherin, is found, which can effectively represent the hepatic endoderm cells that are firstly generated and are AFP⁺ in the differentiation process. Accordingly, hepatic endoderm cells can be separated and purified from miscellaneous human embryonic stem cells by using a flow cell sorting method. The hepatic endoderm cells of the invention show clonal growth, and unlike the previously reported hepatic endoderm cells, they exhibit a strong proliferation ability. Hepatic progenitor cells can be generated by continuously culturing these hepatic endoderm cells. These hepatic progenitor cells also exhibit two differentiation potentials *in vitro,* i.e. the potentials to differentiate into hepatic parenchyma and to differentiate into bile duct. After induction, hepatic progenitor cells can differentiate into hepatocyte-like cells, express their specific function proteins such as ALB, AAT, etc., and store glycogen; hepatic progenitor cells can also differentiate into bile duct-like cells, express KRT7 and KRT19, form a bile duct-like structure, and generate epithelium polarity.

Therefore, the invention, in the third aspect, provides:
1. Hepatic progenitor cells obtained by the differentiation of human embryonic stem cells or human induced pluripotent stem cells, wherein the hepatic progenitor cells express α-fetoprotein, keratin 19 and keratin 7, and possess a proliferation ability and a dual-directional differentiation potential towards hepatocyte-like cells and cholangiocyte-like cells.
2. Hepatic progenitor cells according to above Item 1, **characterized in that**: said human embryonic stem cell is a human embryonic stem cell line.
3. Hepatic progenitor cells according to above Item 1, **characterized in that**: said human embryonic stem cell line is any cell line of: BG01, BG02, BG03, BG04, SA01, SA02, SA03, ES01, ES02, ES03, ES04, ES05, ES06, TE03, TE32, TE33, TE04, TE06, TE62, TE07, TE72, UC01, UC06, WA01, WA07, WA09, WA13 and WA14; the accession numbers are NIH numbers.
4. A method for preparing any one of the hepatic progenitor cells according to the above Items 1-3, comprising the steps of:
   1) culturing human embryonic stem cells or induced pluripotent stem cells on endoderm inducing medium I;
   2) culturing the cells obtained in step 1) on endoderm inducing medium II;
   3) culturing the cells obtained in step 2) on endoderm inducing medium III;
   4) culturing the cells obtained in step 3) on hepatic endoderm cell inducing medium, so as to generate hepatic endoderm cells;
   5) culturing the hepatic endoderm cells with the hepatic progenitor cell culture medium on STO cells as the feeder layer, so as to generate hepatic progenitor cells;
      the endoderm inducing medium I is a basic cell culture medium containing 0.02%-1% w/w of bovine serum albumin component V and 50-200ng/ml human activin A; the endoderm inducing medium II is a basic cell culture medium containing 0.02%-1% w/w of bovine serum albumin component V, 0.05%-0.5% v/v of insulin-transferrin-sodium selenite mixed supplementary liquid and 50-200ng/ml human activin A; the endoderm inducing medium III is a basic cell culture medium containing 0.02%-1% w/w of bovine serum albumin component V, 0.5%-2% v/v of insulin-transferrin-sodium selenite mixed supplementary liquid and 50-200ng/ml human activin A; and the hepatic endoderm cell inducing medium is a hepatocyte culture medium containing 20-60ng/ml human fibroblast growth factor -4 and 10-30ng/ml human bone morphogenetic protein -2;
      the hepatic progenitor cell culture medium is a basic cell culture medium containing 5-25 mM HEPES, 0.5%-2% v/v of insulin-transferrin-sodium selenite mixed supplementary liquid, 0.02%-1% w/w of bovine serum albumin component V, 2-20 mMnicotinamide, 0.2-2mM diphosphorylated ascorbic acid, 0.02-0.2µM dexamethasone, and 5-40ng/ml EGF.
5. The preparation method according to the above Item 4, **characterized in that**: said method further comprises a step of sorting the cells expressing the surface protein N-cadherin by using a flow cytometer.
6. The method according to the above Item 4 or 5, **characterized in that**: the basic cell culture medium is MEM, DMEM, BME, DMEM / F12, RPMI1640 or Fischer's.
7. The method according to any of the above Items 4-6, **characterized in that**: in said method, the human embryonic stem cells or induced pluripotent stem cells are cultured on endoderm inducing medium I for 24h; in said method, the cells obtained in step 1) are cultured on endoderm inducing medium II for 24h; in said method, the cells obtained in step 2) are cultured on endoderm inducing medium III for 24h; in said method, the cells obtained in step 3) are cultured on hepatic endoderm cell inducing medium for 5 days.
8. The method according to any of the above Items 4-7, **characterized in that**: said method further comprises a passage step of the hepatic progenitor cells; the method for the passage of the hepatic progenitor cells comprises the steps of digesting said hepatic progenitor cells with trypsin-EDTA solution, and culturing the resultant cells on hepatic progenitor cell culture medium with STO cells as the feeder layer.
9. The method according to any of the above Items 4-8, **characterized in that**: said human embryonic stem cells are commercially available human embryonic stem cell lines;
   the commercially available human embryonic stem cell line is preferably any cell line of: BG01, BG02, BG03, BG04, SA01, SA02, SA03, ES01, ES02, ES03, ES04, ES05, ES06, TE03, TE32, TE33, TE04, TE06, TE62, TE07, TE72, UC01, UC06, WA01, WA07, WA09, WA13 and WA14; the accession numbers are NIH numbers.
10. A culture medium for preparing hepatic progenitor cells from human embryonic stem cells or induced pluripotent stem cells, which consists of endoderm inducing medium I, endoderm inducing medium II, endoderm inducing medium III, hepatic endoderm cell inducing medium and hepatic progenitor cell culture medium, wherein the endoderm inducing medium I is a basic cell culture medium containing 0.02%-1% w/w of bovine serum albumin component V and 50-200ng/ml human activin A; the endoderm inducing medium II is a basic cell culture medium containing 0.02%-1% w/w of bovine serum albumin component V, 0.05%-0.5% v/v of insulin-transferrin-sodium selenite mixed supplementary liquid and 50-200ng/ml human activin A; the endoderm inducing medium III is a basic cell culture medium containing 0.02%-1% w/w of bovine serum albumin component V, 0.5%-2% v/v of insulin-transferrin-sodium selenite mixed supplementary liquid and 50-200ng/ml human activin A; the hepatic endoderm cell inducing medium is a hepatocyte culture medium containing 20-60ng/ml human fibroblast growth factor -4 and10-30ng/ml human bone morphogenetic protein -2; and the hepatic progenitor cell culture medium is basic cell culture medium containing 5-25 mM HEPES, 0.5%-2% v/v of insulin-transferrin-sodium selenite mixed supplementary liquid, 0.02%-1% w/w of bovine serum albumin component V, 2-20 mM niacinamide, 0.2-2mM diphosphorylated ascorbic acid, 0.02-0.2µM dexamethasone, and 5-40ng/ml EGF.
11. Use of the cells according to any of the above Items 1-3, the method according to any of the above Items 4-9, or the culture medium according to Item above 10 in preparation of hepatocyte-like cells or cholangiocyte-like cells.

The inventive hepatic progenitor cells are the cells obtained by differentiation of human embryonic stem cells or induced pluripotent stem cells and expressing early-stage hepatic marker gene, α-fetoprotein (AFP), and the marker genes of bile duct, keratin 19 (KRT19) and keratin 7 (KRT7). These hepatic progenitor cells possess a strong proliferation ability and a dual-directional differentiation potential towards hepatocyte-like cells and cholangiocyte-like cells. The inventive hepatic progenitor cells have the potential to differentiate into hepatic parenchyma or bile duct *in vitro.*

### Description of the Figures

Figure 1 is the detection result of immunofluorescence and RT-PCR for initial differentiation of iPS cells into hepatocytes (H1: differentiated ES cells H1; 3U1: differentiated hAFF-4U-M-iPS-1; 3U2: differentiated hAFF-4U-M-iPS-3. Same as below).
Figure 2 is the detection result of the mature hepatocyte marker molecules AAT and CYP3A4 for differentiated cells.
Figure 3 is the detection result of the glycogen synthesis function for the differentiated cells, wherein
a represents human hepatocytes; b, c, d represent the differentiated ES cells H1, hAFF-4U-M-iPS-1 and hAFF-4U-M-iPS-3, respectively; e represents the feeder cells; f, g, h represent the ES cells H1, hAFF-4U-M-iPS-1 and hAFF-4U-M-iPS-3 cells that spontaneously differentiate with no cytokine added, respectively.

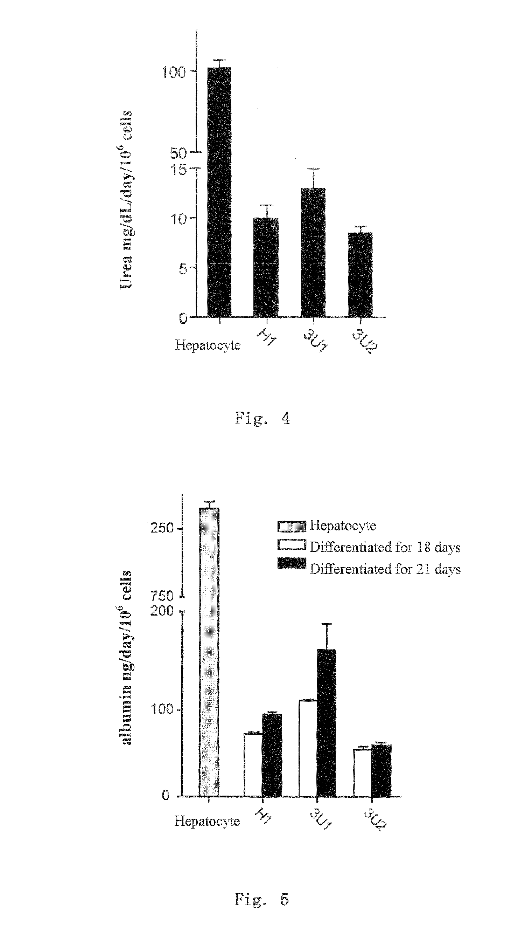

Figure 4 is the result of detection for the urea synthesis function, of the differentiated cells.
Figure 5 is the result of detection for albumin secretion function of the differentiated cells, wherein
control: human hepatocytes; 18: the cells after differentiation for 18 days; 21: the cells after differentiation for 21 days.

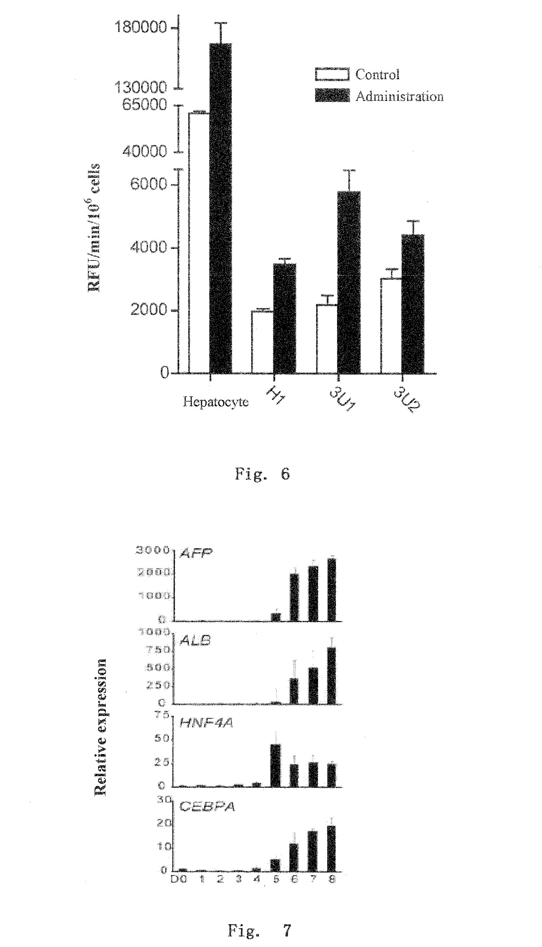

Figure 6 is the detection result of the inducable CYP450 enzyme activity of the differentiated cells, wherein
control: human hepatocytes; administration: 200µg/ml phenobarbital sodium.
Figure 7 shows the time profile of hepatic endoderm related gene expression.

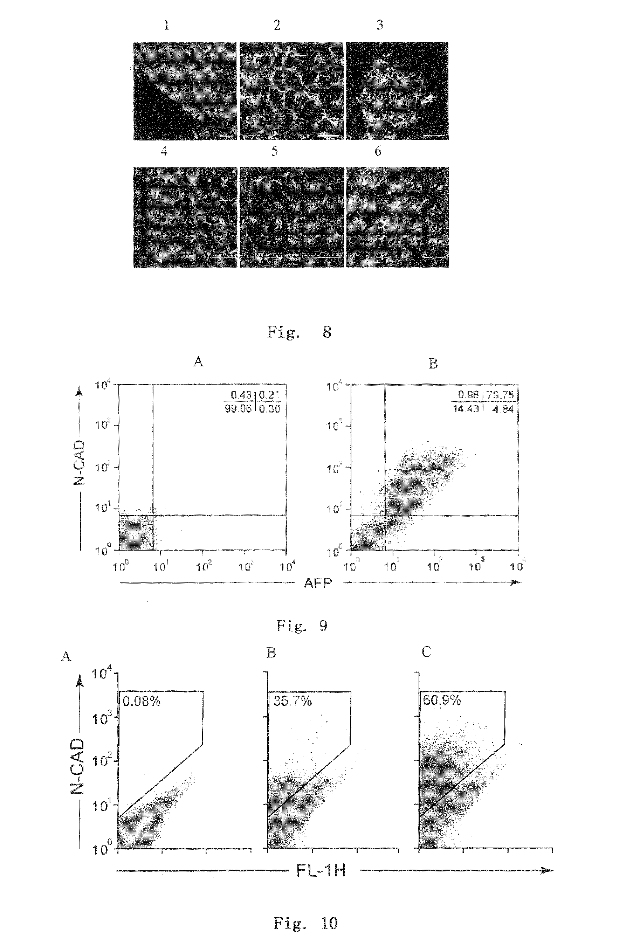

Figure 8 shows the co-expression of N-cadherin with AFP, ALB, HNF4A, GATA4 and FOXA2 as indicated by immunofluorescence, wherein
   1: co-expression of AFP and N-cadherin (AFP in green, N-cadherin in red); 2: co-expression of AFP and N-cadherin (AFP in red, N-cadherin in green); 3: co-expression of ALB and N-cadherin; 4: co-expression of HNF4A and N-cadhein; 5: co-expression of GATA4 and N-cadherin; 6: co-expression of FOXA2 and N-cadherin.
Figure 9 is the result of intracellular flow cytometry, showing the co-expression of N-cadherin and AFP in the same cell, wherein
   A: isotype antibody control; B: the expressions of N-cadherin and α-fetoprotein in hepatic endoderm cells,
Figure 10 is the result of sorting by N-cadherin the cells that differentiate for 8 days, wherein
   A: digested with trypsin; B: digested with trypsin and EDTA; C: digested with trypsin and calcium ion.

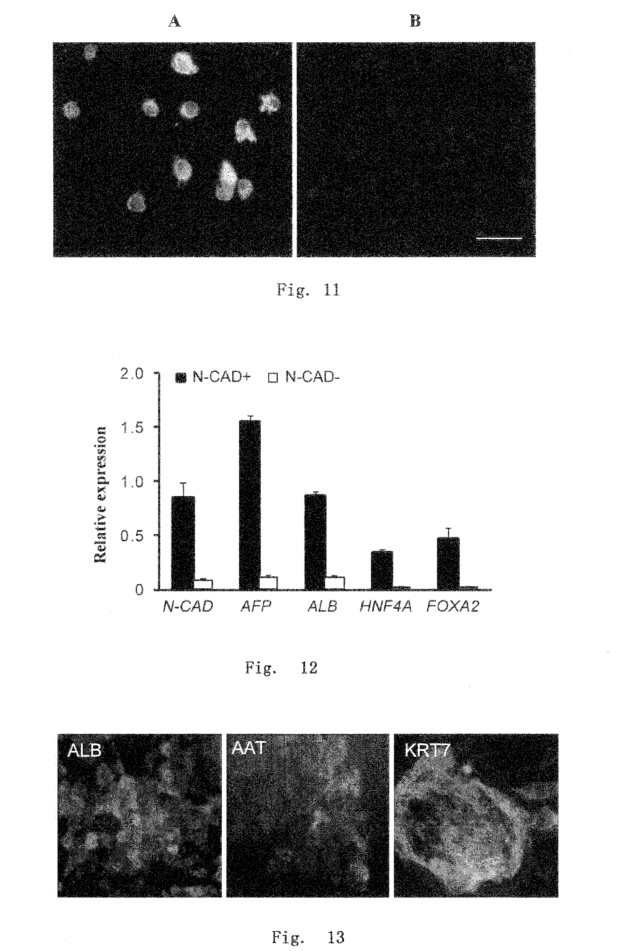

Figure 11 shows the AFP expression of the sorted N-cadherin+ cell population and N-cadherin- cell population, wherein
   A: N-cadherin+ cell population; B: N-cadherin-cell population.
Figure 12 is the result of quantitative RT-PCR, showing the sorted N-cadherin+ cell population is enriched with hepatic specific protein.
Figure 13 shows that the N-cadherin+ cells possess the ability of further differentiation into ALB and AAT positive hepatic parenchyma-like cells and the ability of differentiation into KRT7 positive cells.

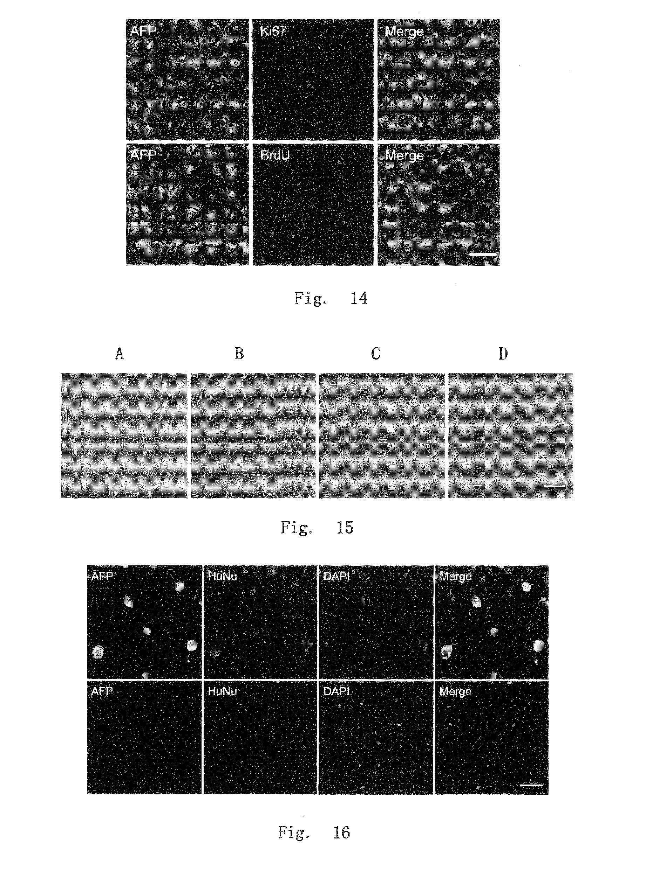

Figure 14 shows that hepatic endoderm cells only have a relatively weak proliferation ability.
   Upper row: only a few hepatic endoderm cells express Ki67; bottom row: only a few hepatic endoderm cells are co-stained with BrdU. Note that most AFP+ cells are BrdU negative. Cellular nuclei are counterstained with DAPI (in blue). Scale, 50µm.
Figure 15 shows the corresponding morphological changes of hepatic progenitor cells,
   A: human embryonic stem cells; B: definitive endoderm cells; C: hepatic endoderm cells; D: hepatic progenitor cells.
Figure 16 shows the specific staining of human cellular nuclei.
   The clones on the STO feeder layer (upper row) are originated from human cells. The bottom row: STO feeder layer does not express human cellular nuclear antigen. Cellular nuclei are counterstained with DAPI (in blue). Scale, 50µm.

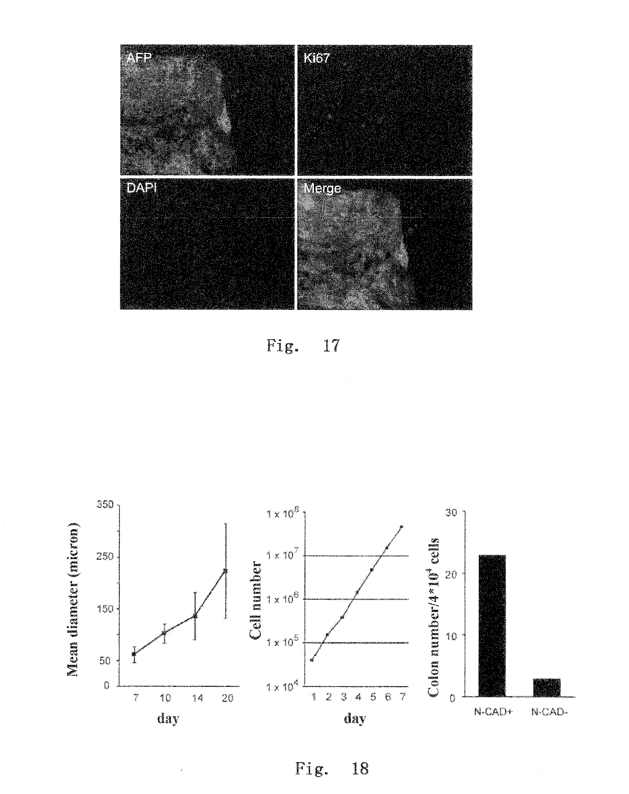

Figure 17 shows that most cells of hepatic progenitor cell clones express Ki67.
Nuclei are counterstained with DAPI (in blue). Scale, 50µm.
Figure 18 shows the proliferation ability of the hepatic progenitor cells.

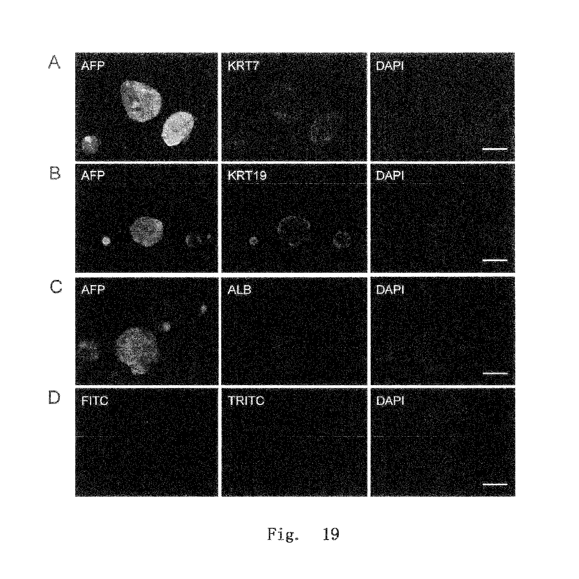

Figure 19 shows the gene expression profile of the hepatic progenitor cells.

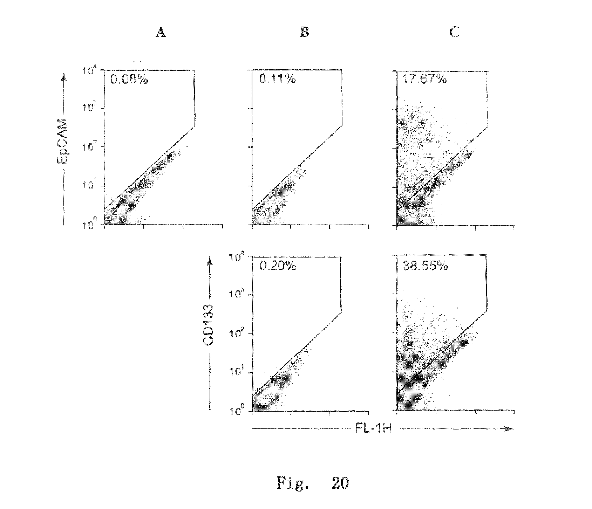

Figure 20 shows EpCAM and CD133 expression of the hepatic progenitor cells as indicated by flow cytometry.
   A: isotype control; B: STO cell control; C: hepatic progenitor cells.

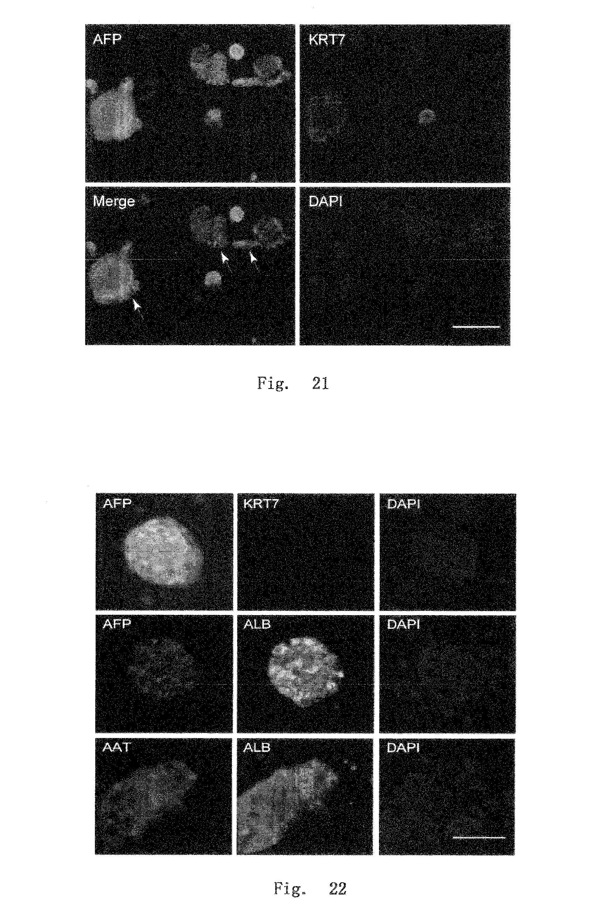

Figure 21 shows that hepatic progenitor cells are capable of differentiating into hepatocytes spontaneously.
Figure 22 shows the directed-induction of differentiation of hepatic progenitor cells into hepatocytes.

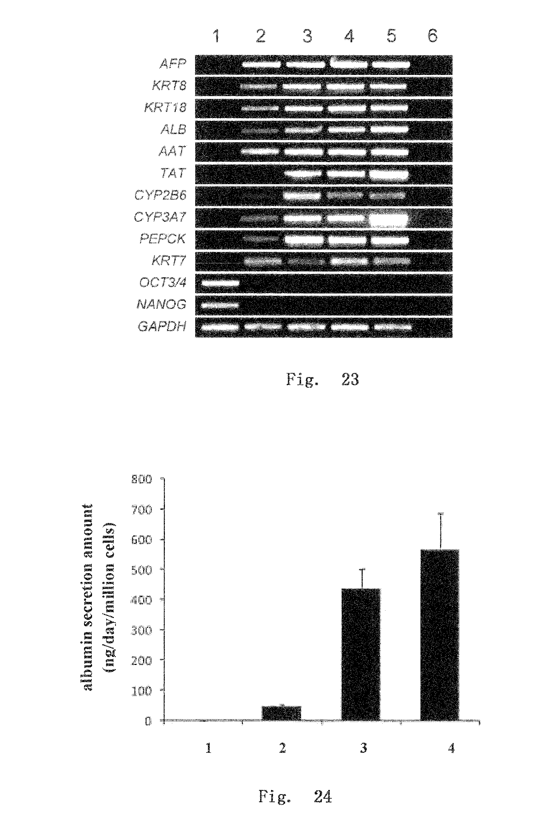

Figure 23 shows mRNA expression of the hepatocytes obtained by differentiation of hepatic progenitor cells.
Figure 24 shows the secretion of human albumin as detected by ELISA, wherein 1: culture medium; 2: hepatic progenitor cells obtained by differentiation of human embryonic stem cells; 3: hepatocytes obtained via differentiation of hepatic progenitor cells; 4: hepatocytes obtained directly from differentiation of human embryonic stem cells.

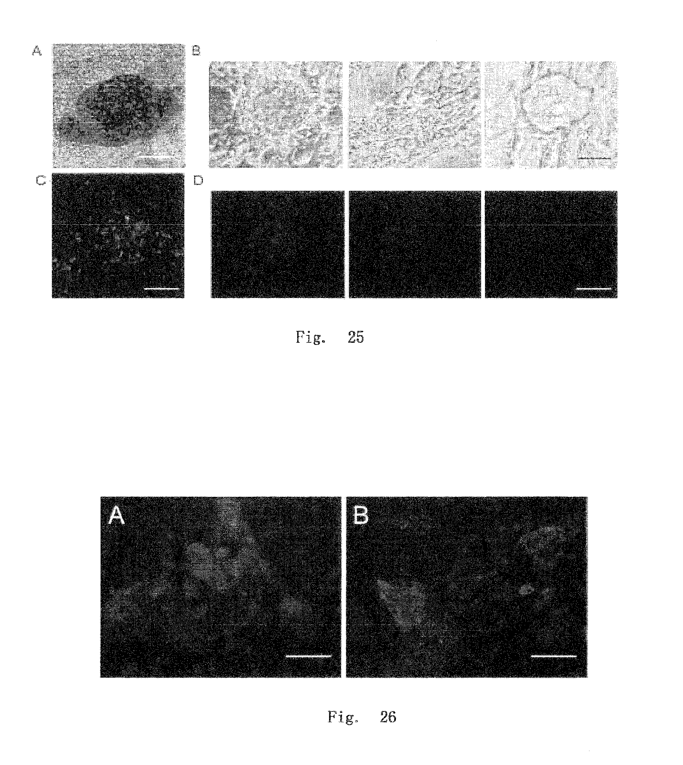

Figure 25 shows the results of function analysis of hepatocyte-like cells obtained by differentiation of hepatic progenitor cells.
Figure 26 shows KRT7 positive and KRT19 positive cells differentiated from hepatic progenitor cells.

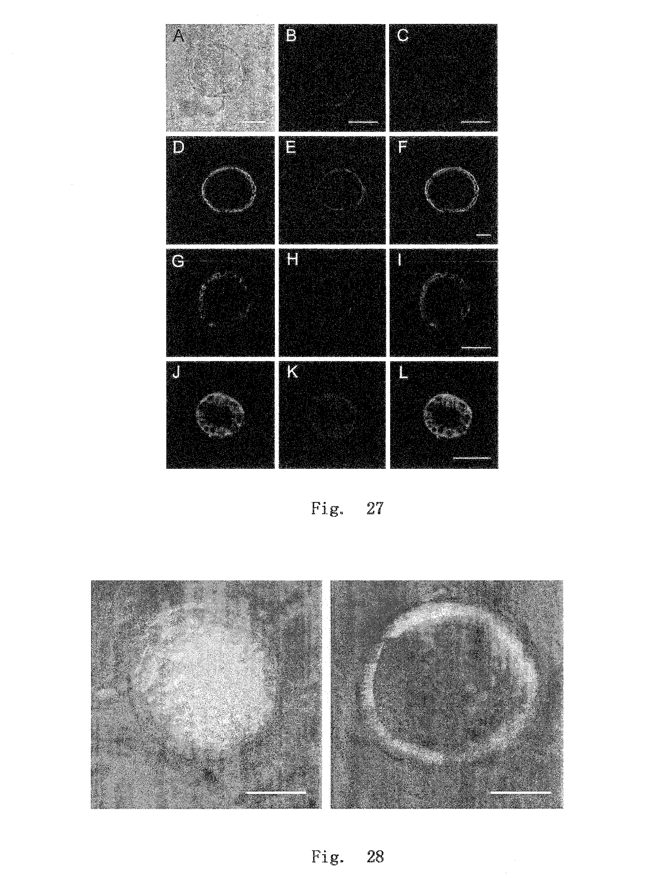

Figure 27 shows the differentiation of hepatic progenitor cells into cholangiocyte-like cells in a three-dimension culture system.
Figure 28 shows the function of the key protein MDR that is involved in bile duct transportation and secretion.
   Left: transportation of rhodamine 123 into the central lumen; right: Verapamil, an inhibitor of MDR, can inhibit the transportation of rhodamine 123. Scale, 50µm,

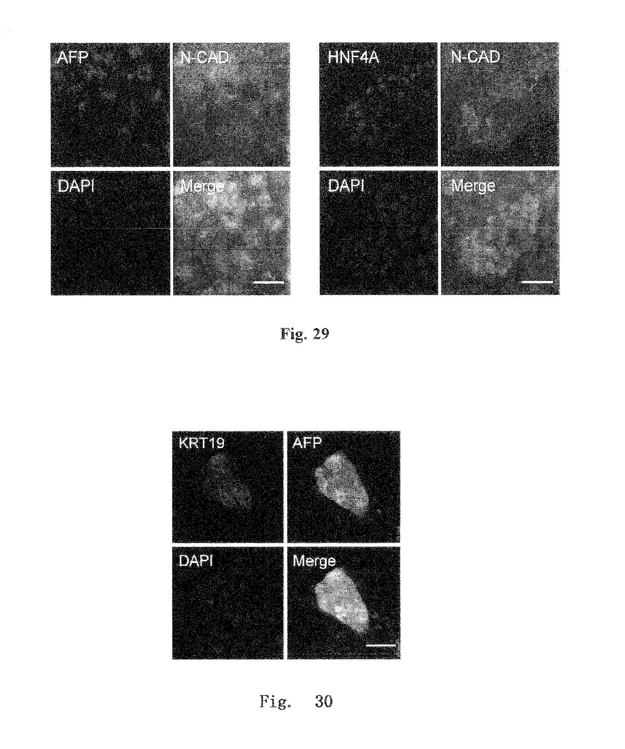

Figure 29 shows the hepatic endoderm cells obtained by differentiation of induced pluripotent stem cells.
   Left: co-expression ofAFP and N-cadherin (AFP in red, N-CAD in green); right: co-expression of HNF4A and N-cadherin (HNF4A in red, N-CAD in green).
Figure 30 shows the hepatic progenitor cells obtained by differentiation of induced pluripotent stem cells,
   wherein AFP is in green, and KRT19 is in red.

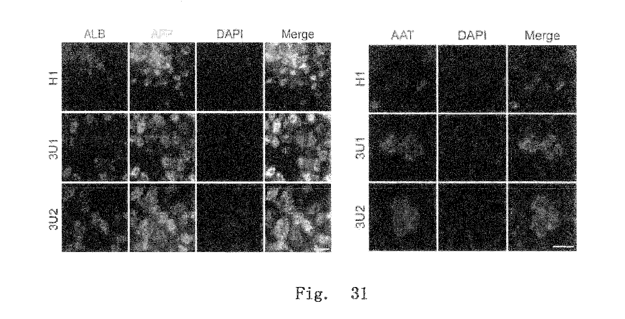

Figure 31 shows that induced pluripotent stem cells further differentiate into hepatic parenchymal cells. H1: human embryonic hepatocyte lines; 3U1 and 3U2: induced pluripotent stem cell lines hAFF-4U-M-iPS-1 and hAFF-4U-M-iPS-3.

### Detailed embodiments of the Invention

The invention will be further described in detail by referring to the Examples. These Examples are only intended to illustrate the invention without limiting the scope of the invention. The scope of the invention is defined by the attached claims.

In the following Examples, the methods used are conventional method unless indicated otherwise, and all of the reagents used are commercially available. Among others, bovine serum albumin component V (Calbiochem Co. USA, 126579), human activin A (Activin A, Peprotech Co. USA, 120-14E), insulm-transferrin-sodium selenite mixed supplementary liquid (Invitrogen Co. USA, 51300-044), HCM MEDIUM(Lonza Co. USA, CC-3198), human fibroblast growth factor -4(FGF4, Peprotech Co. USA, 100-31), human bone morphogenetic protein -2(BMP2, Peprotech Co. USA, 120-02), HEPES (Calbiochem Co. USA, 391338), nicotinamide (Sigma-aldrich Co. USA, N0636-100G), ascorbic acid (Asc-2p, Sigma-aldrich Co. USA, 49752-10G) and EGF(R&D Co. USA, 236-EG-200).

In these Examples, the corresponding cells obtained from human embryonic stem cell lines H1 (NIH accession number: WA01) are substantially same as the cells obtained from human embryonic stem cell lines H7 (NIH accession number: WA07) and from human embryonic stem cell lines H9 (NIH accession number: WA09), respectively, which means that no substantial difference exists,

### Example 1. Induction and detection of differentiation of human ES cells or iPS cells into hepatocytes

### I. Conventional cultivation of human ES cells or iPS cells

### (1) Reagents

PBS: 8g NaCl, 0.2g KCl, 1.44g Na₂HPO₄ and 0.24g KH₂PO₄ were weighted; to which ddH₂O (double distilled water) was added to reach a final volume of 1000mL; and pH value was adjusted to 7.4 by HCl.

2M β- mercaptoethanol (20000x): 1mL of 14.3M β- mercaptoethanol was diluted with 6.15mL PBS, and sterilized by filtering.

human iPS cell culture medium (HESM): 20% Serum Replacement (Knock-out Serum Replacement, KSR), 1mM glutamine (Gibco Co. USA), 0.1mM β- mercaptoethanol, 1% nonessential amino acid (Non-essential AminoAcids, (Gibco Co. USA), and 10ng/mL basic fibroblast growth factor (bFOF) were mixed in DMEM/F12(Invitrogen Co. USA) to a final volume of 1000mL.

0.5mg/mL Dispase (Gibco Co. USA): 10mg Dispase powder was weighted and dissolved in 20mL DMEM/F12 medium, then sterilized by filtering.

1mg/mL collagenase IV (Gibco Co. USA): 20mg collagenase IV powder was weighted and dissolved in 20mL DMEM/F12 medium, then sterilized by filtering.

MEF medium: DMEM(Gibco Co. USA) containing 10%fetal bovine serum.

mitomycin C working fluid: 2mg mitomycin C was dissolved in 200mL DMEM, containing 10% fetal bovine serum at a final concentration of 10µg/mL, then sterilized by filtering.

0.1% gelatin: 0.1g gelatin powder was weighted and dissolved in 1 00mL ddH₂O, then sterilized by autoclaving.

### (2) Obtainment of feeder layer

Mouse embryonic fibroblast (MEF) was treated by the following method, so as to be used as the feeder layer for culturing human iPS cells:
1) adherent MEF cells in good growth state were taken and the MEF medium was discharged, then mitomycin C working fluid containing 10 µg/mL mitomycin C was added to the adherent MEF cells;
2) cultivation was performed at 37°C for 3 hours, during which period the culture dish intended to inoculate MEF cells was treated with 0.1% gelatin and kept at room temperature for 2 hours or longer (or at 37°C for 30 min or longer), and gelatin solution was pipetted off before use;
3) MEF cells were recovered and the mitomycin C working fluid was discharged, the recovered MEF cells were wash 5 times with PBS, so as to remove residual mitomycin completely (because mitomycin is an inhibitor for mitosis, it may result in a toxic effect on IPS cells);
4) digestion was carried out by adding trypsin-EDTA(Gibco Co. USA), and the reaction was terminated with MEF medium;
5) the supernatant was discharged after the sample was centrifuged at 1000rpm for 5 min, and the cell pellet was resuspended in MEF medium and the cell number was counted;
6) the MEF cells treated as above were inoculated into a culture dish coated with 0.1% gelatin at a density of 1.6×10⁵ cells/3.5cm culture dish and kept in an incubator at 37°C for 12-24 hours, so as to obtain the feeder layer used for culturing human ES cells or human iPS cells.

### (3) Conventional cultivation of human ES cells or iPS cells

Human ES cells H1 or iPS cell lines hAFF-4U-M-iPS-1 and hAFF-4U-M-iPS-3 (Yang Zhao, Two supporting factors greatly improve the efficiency of human iPSC generation. Cell Stem Cell, 2008; 3:475-479.) (Peking University) were cultured with human iPS cell culture medium (HESM) on the MEF feeder obtained in step 1. The detailed cultivation method comprises the following steps:
1) the reagents and culture medium (HESM) desired for the cultivation were taken out of the 4°C fridge and preheated at room temperature for approximately 15 min;
2) the cells were washed once with PBS after HESM was pipetted out;
3) the cells were digested by adding 0.5mg/mL Dispase (or 1mg/mL collagenase IV) (1mL/3.5cm culture dish) and cultured in an incubator at 37°C for 10-15 min, then observed under a phase contrast microscope; the digestion was terminated if curved edges appeared at the edges of clones, otherwise the digestion period was extended by returning the cells back into the incubator; during digestion, the cells were checked at any time point, so as to avoid clone shedding caused by over-digestion;
4) after digestion, Dispase or collagenase IV was pipetted out; the cells were washed once with PBS and DMEM/F12 medium respectively, and a suitable amount of DMEM/F12 medium (2mL/3.5cm culture dish) was added;
5) the cell clones were gently scratched off the bottom of the culture dish by a sterile glass dropper with a straight or curved tip, and transferred into a sterile 15mL centrifuge tube with a cone-shaped bottom; the cell clones were gently pipetted several times and then become small cell masses with relatively uniform sizes;
6) after the sample was centrifuged at 1000rpm for 3-4 min, the supernatant was discharged and the cell pellet was resuspended in fresh HESM medium pipetted with a glass dropper;
7) the MEF feeder layer obtained in the above (2) was washed with PBS for 3 times; the small cell masses were inoculated onto the MEF feeder layer, and cultured in an incubator at 37°C for 12-24 hours; after cell adherence, the medium could be replaced with fresh HESM medium; the medium was changed once a day, and each passage usually took 5-7 days; passage must be carried out in time, if (1) the MEF feeder layer has been placed for 2 weeks; (2) cell clones are over-densely or of oversize; or (3) significant spontaneous differentiation of cells appears.

### II. Induction of differentiation of human ES cells or iPS cells into hepatocytes

Differentiation medium I-1: RPMI 1640 medium (Gibco Co. USA) containing 10ng/ml human activin A (Activin A) and 0.01% (volume percentage) insulin-transferrin-selenium salt (sodium selenite) (ITS) mixed supplementary liquid (Gibco Co. USA), pH 7.2-7.6.

Differentiation medium I-2: RPMI 1640 medium (Gibco Co. USA) containing 500ng/ml (human activin A (Activin A) and 20%(volume percentage) ITS, pH 7.2-7.6.

Differentiation medium I-3: RPMI 1640 medium (Gibco Co. USA) containing 100ng/ml human activin A (ActivinA) and 1%(volume percentage) ITS, pH 7.2-7.6.

Differentiation medium I-4: RPMI 1640 medium (Gibco Co. USA) containing 100ng/ml human activin A (Activin A) and 0.1%(volume percentage)ITS, pH 7.2-7.6.

Differentiation medium II-1: hepatocyte culture medium (HCM) (purchased from Cambrex Co.) containing 5ng/ml human fibroblast growth factor (FGF2) and 5ng/ml human bone morphogenetic protein (BMP4)(Peprotech Co. USA), pH 7.2-7.6.

Differentiation medium. II-2: hepatocyte culture medium (HCM) (purchased from Cambrex Co.) containing 100ng/ml human fibroblast growth factor (FGF2) and 100ng/ml human bone morphogenetic protein (BMP4)(Peprotech Co. USA), pH 7.2-7.6.

Differentiation medium II-3: hepatocyte culture medium (HCM) (purchased from Cambrex Co.) containing 30ng/ml human fibroblast growth factor (FGF2) and 20ng/ml human bone morphogenetic protein (BMP4)(Peprotech Co. USA), pH 7.2-7.6.

Differentiation medium III-1: HCM medium containing 5ng/ml human hepatocyte growth factor (HGF, Peprotech Co. USA, 100-39) and 5ng/ml human keratinocyte growth factor (KGF, Amgen Co. USA), pH 7.2-7.6.

Differentiation medium III-2: HCM medium containing 100ng/ml human hepatocyte growth factor (HGF) and 100ng/ml human keratinocyte growth factor (KGF), pH 7.2-7.6.

Differentiation medium III-3; HCM medium containing 20ng/ml human hepatocyte growth factor (HGF) and 20ng/ml human keratinocyte growth factor (KGF), pH 7.2-7.6.

Differentiation medium IV-1: HCM medium containing 1ng/ml oncostatin M(OSM) (R&D Co. USA) and 0.05µM dexamethasone (Dex), pH 7.2-7.6.

Differentiation medium IV-2: HCM medium containing 100ng/ml oncostatin M(OSM) (R&D Co. USA) and 1µM dexamethasone (Dex), pH 7.2-7.6.

Differentiation medium IV-3: HCM medium containing 10ng/ml oncostatin M(OSM) (R&D Co. USA) and 0.1µM dexamethasone (Dex), pH 7.2-7.6.

Differentiation medium V-1: basic culture medium containing 0.1%(volume percentage)N2, 0.1% B27, 0.5mM glutamine , 0.1% nonessential amino acid , 0.05 mM β-mercaptoethanol, 1 ng/ml oncostatin M(OSM) and 0.05µM dexamethasone (Dex), pH 7.2-7.6,

Differentiation medium V-2: basic culture medium containing 10%(volume percentage)N2, 20% B27, 2mM glutamine, 10% nonessential amino acid , 0.2 mM β-mercaptoethanol, 100ng/ml oncostatin M(OSM) and 1µM dexamethasone (Dex), pH 7.2-7.6.

Differentiation medium V-3: basic culture medium containing 1%(volume pereentage)N2, 2% B27, 1mM glutamine, 1% nonessential amino acid , 0.1 mM β-mercaptoethanol, 10ng/ml oncostatin M(OSM) and 0.1µM dexamethasone (Dex), pH 7.2-7.6.

The induction of differentiation of human IPS cells or ES cells into hepatocytes comprised the following steps:
1) induction towards definitive endoderm cells: after the medium was discharged, human IPS cells or ES cells H1 obtained as above and cultured on the MEF feeder layer were washed with PBS twice; differentiation medium I-1, differentiation medium I-2, differentiation medium I-3 or differentiation medium 1-4 was added; the cells were cultured in a cell incubator at 37°C for 1 day (24 hours); then the medium was discharged and replaced with differentiation medium I-1, differentiation medium I-2, differentiation medium I-3 or differentiation medium I-4 containing 0.1% (volume percentage) insulin-transferrin-selenium salt (ITS) (Gibco Co. USA); after cultivation under the same condition for 1 day, the medium was discharged and replaced with differentiation medium I-1 or differentiation medium 1-2 containing 1%(volume percentage) ITS; and cultivation was carried out under the same condition for 1 day;
2) initiation of hepatocyte differentiation: differentiation medium I-1, differentiation medium I-2, differentiation medium I-3 or differentiation medium I-4 containing ITS was discharged; the cells were washed once with PBS; differentiation medium II-1, differentiation medium II-2 or differentiation medium II-3 was added; the cells were cultured in a cell incubator at 37°C for 4 days with medium refreshed once a day, so as to obtain differentiated IPS cells or ES cells;
3) proliferation of differentiated IPS cells or ES cells: differentiation medium II-1, differentiation medium II-2 or differentiation medium II-3 was discharged; the cells were washed once with PBS; differentiation medium III-1, differentiation medium III-2 or differentiation medium III-3 was added; the cells were then cultured in a cell incubator at 37°C for 6 days with medium refreshed once a day;
4) promotion of maturation of differentiated IPS cells or ES cells; differentiation medium III-1, differentiation medium III-2 or differentiation medium III-3 was discharged; the cells were washed with PBS once; differentiation medium IV-1, differentiation medium IV-2 or differentiation medium IV-3 was added; the cells were then cultured in a cell incubator at 37°C for 5 days with medium refreshed once a day; differentiation medium IV-1, differentiation medium IV-2 or differentiation medium IV-3 was discharged; the cells were washed once with PBS; differentiation medium IV-1, differentiation medium IV-2 or differentiation medium IV-3 was added, the cells were then cultured in a cell incubator at 37°C for 3 days with medium refreshed once a day.

### III. Detection of the initial differentiation of IPS cells or ES cells into hepatocytes

### (1) immunofluorescence staining detection

PBST: PBS solution containing 0.2% (volume percentage) Triton X100.

Blocking liquid: PBST solution containing 2% goat serum (or horse serum).

Secondary antibody dilution (0.1% BSA solution): 0.1g bovine serum albumin (BSA) dissolved in 1 00mL PBS.

The ES cells and iPS cells that had been induced to differentiate for 7 days expressed early-stage hepatocyte marker molecules AFP, Alb(ALB) and CK18.

The differentiation state of the cells obtained in step II 2) was detected by a immunofluorescence staining method, and the detection method comprised the following steps:
1) the medium was discharged and the cells were washed with PBS twice;
2) after 4% paraformaldehyde was added, the cells were fixed at room temperature for 15 min (or after absolute methanol was added, the cells were fixed at room temperature for 5-10 min);
3) the cells were washed with PBS for 3 times, 5 min each time;
4) the cells were permeabilized with PBST solution at room temperature for 10 min;
5) the cells were washed with PBS for 5 min once;
6) the blocking liquid was added, and the cells were blocked at room temperature for 30-60 min;
7) the blocking liquid was discharged, and primary antibody (Polyclonal Rabbit Anti-Human Alb, purchased from DAKO Co.), mouse anti-human α-Fetoprotein(AFP) monoclonal antibody (Beijing Zhongshanjinqiao Biotech. Co. Ltd.), mouse anti-human cytokeratin 18 (CK18) monoclonal antibody (Beijing Zhongshanjinqiao Biotech. Co. Ltd.), rabbit anti-human AAT monoclonal antibody (Beijing Zhongshanjinqiao Biotech. Co. Ltd.) or rabbit anti-human CYP3A4 polyclonal antibody (purchased from Serotec Co.) was added; the cells werer kept at 4 °C for 12-24 hours (or incubated at 37°C for 2 hours); wherein the above antibodies were diluted with the blocking solution at a ratio of 1:50;
8) the cells were washed with PBS for 3 times, 5 min each time;
9) secondary antibody (FITC or TRITC labled goat anti-mouse IgG or TRITC(tetraethyl rhodamine isothiocyanate) labeled goat anti-rabbit IgG) (Beijing Zhongshanjinqiao Biotech. Co. Ltd.) (the secondary antibody was diluted in the secondary antibody diluent at a ratio of 1:50-150) was added; and the cells were kept at 4 °C in dark for 12 hours (or at 37°Cin dark for 1 hour);
10) the cells were washed with PBS for 3 times, 5 min each time;
11) DAPI ((4',6-diamidino-2-phenylindole) solution (Roche Co. USA) at a final concentration of 1mg/mL was added; and the cells were kept at room temperature for 5 min;
12) the cells were washed with PBS for 3 times, 5 min each time;
13) 500µl PBS (or PBS: glycerol (1:1, v/v)) was added, and the cells were observed and photographed under a fluorescent microscope.

### (2) RT-PCR detection

The iPS cells or ES cells that had differentiated for 7 days were treated with Trizol (Invitrogen Co. USA) so as to extract total RNA from the samples. cDNA was obtained by reverse transcription (the reverse transcription kit from promega Co. USA). PCR identification was performed by using the cDNA as the templet. The primers are shown as follows:
AFP sense primer: TTTTGGGACCCGAACTTTCC; (SEQ ID No: 1)
AFP antisense primer: CTCCTGGTATCCTTTAGCAACTCT. (SEQ ID No: 2)
A1b sense primer: GGTGTTGATTGCCTTTGCTC; (SEQ ID No: 3)
Akb antisense primer: CCCTTCATCCCGAAGTTCAT. (SEQ ID No: 4)
CK8 sense primer: GGAGGCATCACCGCAGTAC; (SEQ ID No: 5)
CK8 antisense primer: TCAGCCCTTCCAOCCGAGAC. (SEQ ID No: 6)
CK18 sense primer: GGTCTGGCAGGAATGGGAGG; (SEQ ID No: 7)
CK18 antisense primer: GGCAATCTGGGCTTGTAGGC. (SEQ ID No: 8)
HNF4α sense primer: CCACGGGCAAACACTACGG: (SEQ ID No: 9)
HNF4α antisense primer: GGCAGGCTGCTGTCCTCAT. (SEQ ID No: 10)
GAPDH sense primer: AATCCCATCACCATCTTCC; (SEQ ID No: 11)
GAPDH antisense primer: CATCACGCCACAGTTTCC. (SEQ ID No: 12)
CK19 sense primer: AATAAATAGGATCCATGCAG;
CK19 antisense primer: TTTTAATGAATTCAGTAGAT.

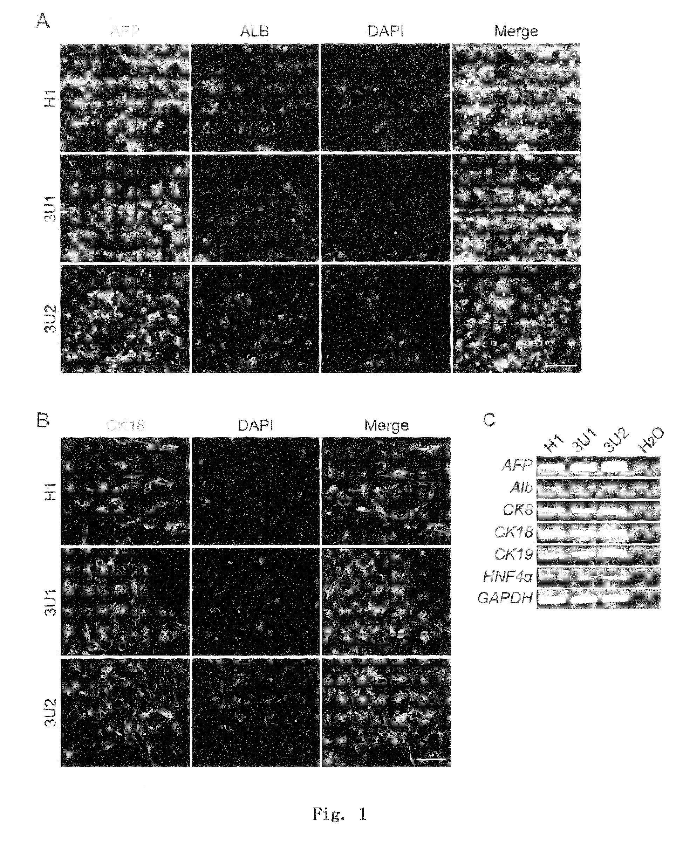

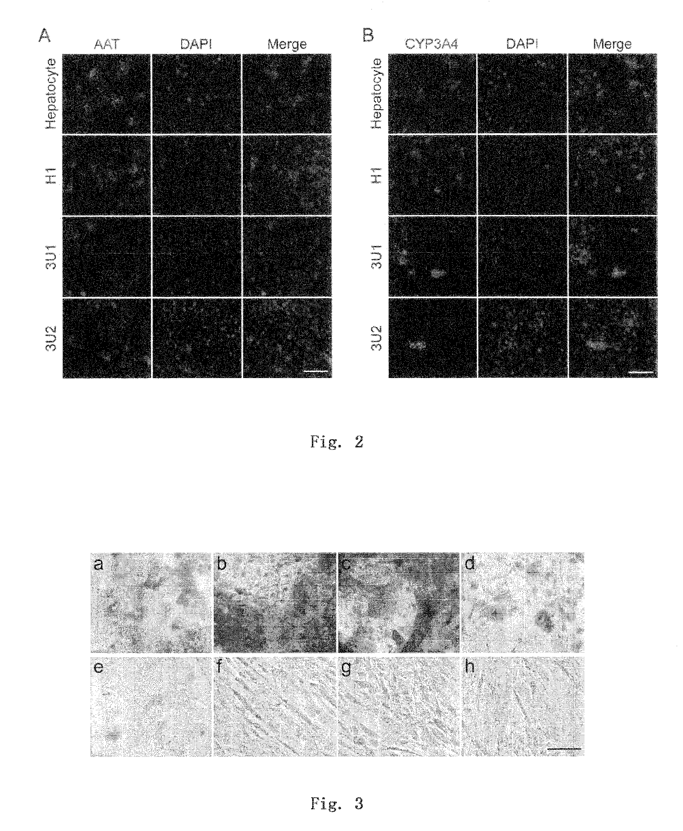

The differentiated iPS cells and ES cells expressed hepatocyte marker molecules AFP, Alb, CK18, AAT and CYP3A4, and the result of the RT-PCR detection also showed that the iPS cells and ES cells that had differentiated for 7 days expressed hepatocyte marker molecules AFP, Alb, CK8, CK18, CK19, HNF4α and GAPDH(glyceraldehyde-3-phosphate dehydrogenase) (Figures 1 and 2).

### IV. Detection of glycogen synthesis function of hepatocytes

The detection was performed by PAS staining method (Periodic Acid-Schiff Stain). The detailed procedure is shown in the instructions of the kit for detecting glycogen synthesis function of hepatocytes (Sigma Co. USA).

The differentiated iPS cells and ES cells had a glycogen synthesis and storage function similar to that of hepatocytes (Figure 3).

### V. Detection of urea synthesis function of hepatocytes

This detection was carried out with a kit for detecting nitrogen in urea. The detailed procedure is shown in the instructions (STANBIO Co. USA).

The differentiated iPS cells and ES cells had a similar urea synthesis function with that of hepatocytes (Figure 4).

### VI. Detection of albumin secretion function of hepatocytes

This detection was carried out with ELISA kit. The detailed procedure is shown in the instructions (Bethyl Co. USA).

The differentiated iPS cells and ES cells had a similar albumin secretion function with that of hepatocytes (Figure 5).

### VII. Detection of CYP450 enzymatic activity of hepatocytes

This detection was carried out with CYP450 fluorescence detection kit. The detailed procedure is shown in the instructions (Sigma Co. USA).

The differentiated iPS cells and ES cells had a drug-induced P450 enzyme activity similar with that of hepatocytes (Figure 6).

The above results indicated that human iPS cells were induced to differentiate into hepatocytes.

### Example 2. Preparation and Identification of hepatic endoderm cells

### I. Obtainment of hepatic endoderm cells

### Day 1:

1) induction of human embryonic stem cells H1, H7 or H9 started at 2-3 days after passage, and the cells in good growth state were selected to be subjected to the differentiation experiment;
2) human embryonic stem cell culture medium (i.e. basic cell culture medium DMEM/F 12 supplemented with 20% serum replacement (Knock-out Serum Replacement, KSR, Invitrogen Co. USA, 10828028), 1mM glutamine (Invitrogen Co. USA, 25030-081), 0.1mM β- mercaptoethanol (Invitrogen Co. USA, 21985-023), 1% nonessential amino acid (Non-essential AminoAcids)( Invitrogen Co. USA, 11140-076), 4ng/mL basic fibroblast growth factor (bFGF, Peprotech Co. USA, 100-18B)) was discharged, and the cells were washed twice with PBS;
3) endoderm inducing medium I, i.e. RPMI1640 medium supplemented with bovine serum albumin component V (Calbiochem Co. USA, 126579) and human activin A (Activin A, Peprotech Co. USA, 120-14E), pH 7.2-7.6, was added; in the endoderm inducing medium I, bovine serum albumin component V had a final concentration of 0.05% (weight percentage), and human activin A (Activin A) had a final concentration of 100ng/ml.

### Day 2:

1) the medium of Day 1 was discharged and replaced with endoderm inducing medium II, i.e. RPMI1640 medium supplemented with bovine serum albumin component V (Calbiochem Co. USA, 126579), human activin A (Activin A) and insulin-transferrin-sodium selenite mixed supplementary liquid (Invitrogen Co. USA, 51300-044), pH 7.2-7.6; in the endoderm inducing medium II, bovine serum albumin component V had a final concentration of 0.05% (weight percentage), human activin A (Activin A) had a final concentration of 100ng/ml, insulin-transferrin-sodium selenite mixed supplementary liquid had a final concentration of 0.1% (volume percentage).

### Day 3:

1) the medium of Day 2 was discharged and replaced with endoderm inducing medium III, i.e. RPMI1640 medium supplemented with bovine serum albumin component V, human activin A (Activin A) and insulin-transferrin-sodium selenite mixed supplementary liquid (Invitrogen Co. USA, 51300-044), pH 7.2-7.6; in the endoderm inducing medium III, bovine serum albumin component V had a final concentration of 0.05% (weight percentage), human activin A (Activin A) had a final concentration of 100ng/ml, insulin-transferrin-sodium selenite mixed supplementary liquid had a final concentration of 1% (volume percentage).

### Day 4-8: the following steps were repeated every day:

1) the medium of the previous day was discharged, and cells were washed with PBS once;
2) the cells were cultured with the added hepatic endoderm cell inducing medium.

Hepatic endoderm cells were obtained on Day 8. Hepatic endoderm cell inducing medium is HCM medium(Lonza Co. USA, CC-3198) supplemented with human fibroblast growth factor -4 (FGF4, Peprotech Co. USA, 100-31) and human bone morphogenetic protein -2(BMP2, Peprotech Co. USA, 120-02), pH 7.2-7.6. In the hepatic endoderm cell inducing medium, human fibroblast growth factor -4(FGF4) had a final concentration of 30ng/ml, and human bone morphogenetic protein -2(BMP2) had a final concentration of 20ng/ml,

The expression time dynamic profile of early-stage liver related genes such as *AFP*, *ALB(i.e. Alb), HNF4A, CEBPA,* etc. was detected by a RT-PCR method.
Primers (from left to right: 5' to 3' direction):
AFP: upstream CCCGAACTTTCCAAGCCATA (SEQ ID No: 13).
   downstream TACATGGGCCACATCCAGG (SEQ ID No: 14);
ALB: upstream GCACAGAATCCTTGGTGAACAG (SEQ ID No: 15),
   downstream ATGGAAGGTGAATGTTTCAGCA (SEQ ID No: 16);
HNF4A: upstream ACTACATCAACGACCGCCAGT (SEQ ID No: 17),
   downstream ATCTGCTCGATCATCTGCCAG (SEQ ID No: 18);
CEBPA: upstream ACAAGAACAGCAACGAGTACCG (SEQ ID No: 19),
   downstream CATTGTCACTGGTCAGCTCCA (SEQ ID No: 20).

All of the genes *AFP*, *ALB*, *HNF4A, CEBPA* showed a similar expression pattern during differentiation. That is, the expression started on -Day 5, and reached the maximum on Day 8 (Figure 7), which indicated that hepatic endoderm cells had been generated.

In the differentiated product of human embryonic stem cells, N-cadherin was specifically expressed in all and only the cells that expressed AFP. The experiment was repeated. By observation under a confocal microscope, the specificity of co-expression of N-cadherina and AFP was confirmed (Figure 8). In Figure 8, panel was photographed under a fluorescent microscope, and the other panels were photographed under a confocal microscope. The scale is 50µm. Cellular nuclei were counterstained with DAPI (Roche Co. USA, 10236276001) (in blue).

The intracellular flow cytometry also showed a similar result, i.e. N-cadheim and AFP, were expressed in same cells (Figure 8). It was confirmed in a further immunofluorescent experiment that N-cadherin was also co-expressed with other hepatic endoderm marker proteins such as ALB, HNF4A, FOXA2, GATA4, etc, which indicates that N-cadherin is a surface marker protein specific to hepatic endoderm,

N-cadherin is a calcium-dependent cell-cell attachment protein, which is highly sensitive to trypsin treatment. However, calcium ions can protect N-cadherin against being digested by trypsin (Yoshida and Takeichi, Cell. 1982 Feb; 28(2):217-24.). When the hepatic endoderm cells that had differentiated for 8 days were digested with conventional trypsin-EDTA solution (Invitrogen Co. USA, 25200114), many extracellular domains of N-cadherin were lysed by trypsin. As a result, N-cadherin protein cannot be identified by the N-cadherin antibody (clone No. GC4, purchased form Sigma-Aldrich Co. USA) used in the flow sorting (Figure 10B). If hepatic endoderm was treated with EDTA-free trypsin (Invitrogen Co. USA, 27250018) with 2mM calcium ions added at the same time, the integrity of N-caherin protein could be effectively protected (Reiss et al., EMBO J. 2005 Feb 23; 24,742 - 752).

N-cadherin⁺ cell population was separated by flow cytometer; and N-cadherin cell population was simultaneously collected as a control.

N-cadheim positive cell population was sorted from the differentiation product of Day 8 by flow sorting (60.9%±9.1%, Figure 10C).

It was shown by immunocytochemistry of the sorted N-cadherin cells that more than 90% cells in the N-cadheirn⁺ cell population expressed AFP; whereas almost no AFP positive cell existed in the N-cadherin cell population (Figure 11). The N-cadherin+ cell population expressed AFP (in green).

Moreover, by quantitative RT-PCR analysis of the sorted cells, it was found that the N-cadheim+ cell population was enriched with hepatic specific expression genes α-fetoprotein (AFP), albumin (ALB), hepatocyte nuclearfactor 4A(HNF4A) and hepatocyte nuclearfactor 3B (FOXA2) (Figure 12). The used upstream and downstream primers are shown as follows:
AFP: upstream CCCGAACTTTCCAAGCCATA (SEQ ID No: 21), downstream TACATGGGCCACATCCAGG (SEQ ID No: 22);
ALB: upstream GCACAGAATCCTTGGTGAACAG (SEQ ID No: 23), downstream ATGGAAGGTGAATGTTTCAGCA (SEQ ID No: 24);
HNF4A: upstream ACTACATCAACGACCGCCAGT (SEQ ID No: 25), downstream ATCTGCTCGATCATCTGCCAG (SEQ ID No: 26);
FOXA2 : upstream CTGAGCGAGATCTACCAGTGGA (SEQ ID No: 27), downstream CAGTCGTTGAAGGAGAGCGAGT (SEQ ID No: 28)).

The result of each quantitative PCR was repeated 3 times. The expression difference between N-cad+ and N-cad⁻ for each gene had a significance of less than 0.01.

### II. Induction of differentiation of hepatic endoderm cells into mature hepatic parenchymal cells

1) the N-cadheirn⁺ cells or N-cadherin⁻ cells obtained in step I were washed once with PBS; hepatic parenchymal cell culture medium I, i.e. HCM MEDIUM containing 20ng/ml human hepatocyte growth factor (HGF, Peprotech Co. USA, 100-39) (Lonza Co. USA, CC-3198), was added, wherein the above steps were repeated once a day, cultivation was performed for 5 days in total;
2) hepatocyte culture medium I was discharged, and the cells was washed once with PBS; hepatic parenchymal cell culture medium II, i.e. HCM medium containing 10ng/ml tumor inhibitor M (OSM, R&D Co. USA, 295-OM-050), 0.1 µM dexamethasone (Sigma-Aldrich Co. USA, D8893) was added.

N-cadheirn⁺ cells could further differentiate into hepatic parenchyma-like cells that expressed ALB and AAT, and bile duct-like cells that expressed KRT7 (Figure 13). The N-cadheirn⁺ cells in Figure 7 had an ability to further differentiate into not only ALB(Figure 13A), AAT(Figure 13B) positive hepatic parenchyma-like cells, but also KRT7(Figure 13C) positive cells.

In contrast, N-cadherin cells could not differentiate into liver and bile duct lineages. The above experiments demonstrated that the N-cadheim⁺ cells were the hepatic endoderm cells differentiated from human embryonic stem cells.

Hepatic endoderm cells could be obtained by differentiation of induced pluripotent stem (iPS) cell lines hAFF-4U-M-iPS-1 and hAFF-4U-M-iPS-3 (Yang Zhao, Two supporting factors greatly improve the efficiency of human iPSC generation. Cell Stem Cell, 2008;3:475-479.) (Peking University) in the same way. These hepatic endoderm cells co-expressed AFP and N-cadherin, and co-expressed HNF4A and N-cadherin (Figure 30). These hepatic endoderm cells also expressed genes ALB, FOXA2, GATA4 etc. Through the method shown as above, these hepatic endoderm cells could also be further induced to differentiate into mature hepatic parenchymal cells which expressed proteins ALB and AAT, etc. (Figure 31), or into KRT7 positive bile duct-like cells.

### Example 3. Preparation of hepatic progenitor cells from the hepatic endoderm cells derived from human embryonic stem cells

### I. Generation of hepatic progenitor cells from hepatic endoderm cells

### A) Obtainment of hepatic progenitor cells

1) the hepatic endoderm cells obtained in Example 2 was washed once with PBS;
2) if N-cadherin sorting was not performed, then digestion was carried out with trypsin-EDTA solution at room temperature for 1 min; if N-cadherin sorting was desired, then digestion was carried out with EDTA-free trypsin (trypsin solution added with 2mM CaCl₂) at 37°C for about half an hour;
3) digestion was terminated by adding DMEM medium containing 10 (v/v) % fetal bovine serum, and the cells were suspended and transferred into a 15ml centrifuge tube;
4) the cells were centrifuged at 1000 rpm for 5 min and resuspended in a hepatic progenitor cell culture medium, i.e. DMEM/F-12 basic culture medium supplemented with HEPES (Calbiochem Co. USA, 391338), insulin-transferrin-sodium selenite mixed supplementary liquid (Invitrogen Co. USA, 51300-044), bovine serum albumin component V, niacinamide (Sigma-aldrich Co. USA, N0636-100G), ascorbic acid (Asc-2p. Sigma-aldrich Co. USA, 49752-10G), dexamethasone and EGF(R&D Co. USA, 236-EG-200), pH 7.2-7.6; in the hepatic progenitor cell culture medium, HEPES had a final concentration of 10mM. insulin-transferrin-sodium selenite mixed supplementary liquid had a final concentration of 1% (volume percentage), bovine serum albumin component V had a final concentration of 0.05% (weight percentage), niacinamide had a final concentration of 11mM, ascorbic acid (Asc-2p) had a final concentration of 1mM, dexamethasone had a final concentration of 0.1µM and EGF had a final concentration of 10ng/ml;
5) hepatic endoderm cells were purified by sorting for N-cadherin by using the same method as Example 2; and N-cadheim⁺ cells were obtained;
6) preparation of STO feeder layer cells: mouse embryonic fibroblast cell line (STO) cells (China Center for Type Cell Culture Collection) that grew well and were ∼90% confluent were treated with 10µg/ml mitomycin C (Roche Co. USA, 10107409001) for 4-6 hours; the culture dish was treated with 0.1% gelatin (Sigma-Aldrich Co. USA, G1890-100G) at 37°C for 30 min or at room temperature for 2 hour; the cells treated with mitomycin C were washed with PBS for 5 times to remove the residual mitomycin C completely; after being digested with trypsin, the cells were inoculated into the culture dish treated with gelatin at a density of 1:3 and cultured overnight for use;
7) the obtained STO feeder layer cells were washed twice with PBS;
8) the N-cadheirn⁺ cells were inoculated onto the STO feeder layer cells, to which hepatic progenitor cell culture medium was added; the cells were cultured in an incubator filled with CO₂;
9) the medium was refreshed every day; and generation of clones was observed clearly on ∼ Day 7-10.

### B) Passage of hepatic progenitor cells

1) the cells obtained in A) was washed once with BPS after the hepatic progenitor cell culture medium was discharged;
2) trypsin-EDTA solution (Invitrogen Co. USA, 25200114) was added to digest the cells at room temperature for 3-5 min; the cells were then observed under a microscope; the cells were ready for use if they contracted into a round shape and were detached from one another;
3) the digestion was terminated by adding DMEM containing 10 (v/v) % fetal bovine serum;
4) the cells were suspended and transferred into a 15ml centrifuge tube, and centrifuged at 1000 rpm for 5 min;
5) the cells were resuspended in hepatic progenitor cell culture medium;
6) the obtained STO feeder layer cells were washed twice with PBS;
7) the cells were then inoculated onto the STO feeder layer cells, to which hepatic progenitor cell culture medium was added;
8) the cells were cultured in an incubator filled with CO₂, with medium refreshed every day.

### C) Maintenance of hepatic progenitor cell cultivation

The cells of step B) were cultured with hepatic progenitor cell culture medium on the STO feeder layer. The medium was refreshed once a day. The cell passage took 7-10 days for each generation. Passage would be carried out in time, if the feeder layer was placed for 2 weeks, or kept till the feeder turned into a poor quality or hepatic progenitor cell clones were over-densely or of oversize.

During the development of embryonic livers, once the liver fate specialization is accomplished, liver buds generate and hepatic progenitor cells start to be dramatically proliferated till reaching the size of the corresponding liver tissues finally. However, by detecting the hepatic endoderm cells derived from human embryonic stem cells, it was found that these hepatic endoderm cells have a very low proliferation ability. The hepatic endoderm cells were detected by AFP and Ki67 immunofluorescence (AFP and Ki67 antibodies were purchased from Zhongshanjinqiao Co.), and the results showed that there barely existed AFP positive cells and Ki67 co-staining (Figure 14). When BrdU was added to the medium during the 5 days of the whole hepatic endoderm generating stage, the results of the detection showed that only less than 5% of the AFP positive cells expressed BrdU (Figure 14). These studies indicated that the hepatic endoderm cells derived from human embryonic stem cells started to differentiate quickly, which resulted in loss of proliferation ability.

Hepatic progenitor cells were generated as follow:

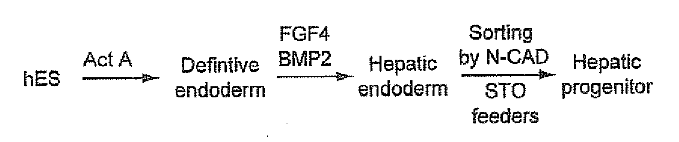

When the hepatic endoderm cells derived from human embryonic stem cells were cultured as above, some parenchymal cell clones could be generated (Figure 15). It was shown in Figure 15 that the human embryonic stem cell clones were in a flat-round shape, and had trim cell edges; the endodermic cells were scaly and flat monolayer cells; the hepatic endoderm cells were in a form of monolayer or multilayer; and the hepatic progenitor cells formed dense clones with trim and smooth edges. The scale was 50µm. These clones had complete and smooth edges. In contrast with the hepatic endoderm cells that could not passage, these clones could be proliferated continuously. The specific immunofluorescence detection of human cellular nuclei (antibodies were purchased from Chemicon Co. USA) showed these cells were derived from human cells, rather than STO cells (Figure 16). Therefore, these clones were the hepatic progenitor cells derived from human embryonic stem cells. Most cells in these clones expressed Ki67 (Figure 17). In order to further demonstrate its proliferation ability, the size change of these clones while growing was studied. On Day 7 after these clones were transferred to the STO feeder layer cells, these hepatic progenitor cells formed clones with a diameter of 62.0±15.4µm. On Day 20 during cultivation, the size of these clones reached 225.4±92.0µm, which showed a slow but real cell proliferation. These hepatic progenitor cells were passaged at a ratio of 1:2 or 1:3, and the cells were cultured *in vitro* for over 12 generations. The cells were frozen and revived repeatedly (Figure 18). As a control, the feeder layer cells that had been treated with mitomycin and cultured separately could not generate clones under the same culture condition.

In Figure 18, left: the number of hepatic progenitor cell clones increased through culture period, and their sizes increased gradually as well, n=3. Middle: the growth curve of hepatic progenitor cells. Right: N-cadherin+ population had a higher ability of generating clones than N-cadherin- population. This experiment was repeated 3 times and provided similar results. The result shown here represented a typical one.

In order to further identify hepatic progenitor cells, expression of α-fetoprotein (AFP), albumin (ALB), cellular keratin 19(KRT19) and cellular keratin 7(KRT7) was detected by an immunofluorescent method (the antibodies against AFP, KRT19 and KRT7 were purchased from Zhongshanjinqiao Co,; the antibody against ALB was purchased from DAKO Co. USA). These hepatic progenitor cells expressed early-stage hepatic marker gene AFP, but weakly expressed or did not express mature hepatocyte marker ALB. These clones also expressed bile duct marker genes KRT19 and KRT7 (Figure 19). Figure 19A, shows that hepatic progenitor cells co-expressed AFP and KRT7; Figure 19B shows that hepatic progenitor cells expressed KRT19; and Figure 19C shows that hepatic progenitor cells expressed ALB. Figure 19D is the negative control, wherein cellular nuclei were counterstained with DAPI (in blue). The scale was 50µm. In addition, they also expressed presumed hepatic progenitor cell markers EpCAM and CD133(Figure 20) (Schmelzer et al., J Exp Med. 2007 Aug 6;204(8):1973-87).

To compare the ability of generating hepatic progenitor cells by N-cadherin⁺ hepatic endoderm cell population and by N-cadherin⁻ cell population after determination of liver fate, N-cadherin⁻ cell population was cultured in the same way. The results showed that the number of clones generated from N-cadherin⁻ cell population was at least 6 folds lower than that of N-cadherin⁺ population (Figure 18), And these clones disappeared rapidly after passage, which indicated their low proliferation ability. So these clones are not hepatic progenitor cells mentioned earlier. This result also demonstrates that N-cadherin can be used as a specific surface marker protein for separating and purifying hepatic endoderm cells from human embryonic stem cell differentiation system, so as to differentiate the generated hepatic progenitor cells.

### II. Differentiation of hepatic progenitor cells into two lineages i.e. liver and bile duct

### A) Differentiation of the hepatic progenitor cells derived from human embryonic stem cells into hepatocyte-like cells

### Day 1:

1) the medium was discharged;
2) the hepatic progenitor cells obtained in step I were washed once with PBS;
3) hepatic parenchymal cell culture medium I, i.e. HCM medium containing 20ng/ml hepatocyte growth factor (HGF) was added.

### Day 2-5: the following step was repeated every day:

4) the medium of the previous day was discharged, and fresh hepatocyte cell culture medium I was added.

### Day 6-10: the following steps were repeated every day:

5) the medium of the previous day was discharged;
6) hepatocyte cell culture medium II, i.e. HCM medium containing 10ng/ml OSM, 0.1 µM dexamethasone was added.

When the hepatic progenitor cells derived from human embryonic stem cells were undergoing proliferation, we found that some cells moved out of the clones from the trim edges. In contrast with AFP⁺KRT7⁺ progenitor cells, the cells at the edges of the clones became AFP⁺KRT7⁻ cells, which meant that they might have differentiated into hepatic parenchymal cells spontaneously (Figure 21). The arrows indicate AFP+KRT7- cells. Cellular nuclei were counterstained with DAPI (in blue). The scale was 50µm.

In order to further confirm the potential to differentiate hepatic progenitor cells into hepatocytes, the directed differentiation of progenitor cells into hepatocytes was promoted with HGF and OSM. Hepatic progenitor cells were first cultured in hepatocyte culture medium (HCM) containing 20ng/ml HGF for 5 days, then continuously cultured in hepatocyte culture medium (HCM) containing 10ng/ml OSM and 0.1µM dexamethasone, for 5 days. The differentiated cells were detected by an immunofluorescent technique for the marker proteins of hepatocytes. The differentiated cell colonies lost KRT7 expression and turned to express ALB, whereas ALB was only weakly expressed in hepatic progenitor cells. Furthermore, these ALB expressing cells also expressed AAT (Figure 22), wherein the hepatic progenitor cells were induced to become KRT7 negative (upper row), ALB (middle and bottom rows) and AAT (bottom row) positive hepatocyte-like cells. Cellular nuclei were counterstained with DAPI (in blue). The scale was 50µm.

It was found in RT-PCR analysis that many genes specific to mature hepatic parenchymal cells, such as ALB, AAT, TAT, KRT8, KRT18, as well as cytochrome P450 family members CYP3A7 and CYP2A6, were expressed in the induced cells (Figure 23). At the same time, the differentiated cells lost the expression of pluripotent marker genes OCT4 and Nanog, which indicated that the differentiated cell population did not include any undifferentiated human embryonic stem cells and might be used for cell transplantation experiments in the future (Figure 23). (Primers are shown in Table 2.)

In Figure 23,1: human embryonic stem cells; 2: hepatic progenitor cells obtained by differentiation of human embryonic stem cells; 3: hepatocytes obtained via differentiation of hepatic progenitor cells; 4: hepatocytes obtained directly by differentiation of human embryonic stem cells; 5: human embryonic hepatocytes; 6: cDNA that was not reverse transcribed.

**Table 2: Sequences of the primers used for detecting gene expression of hepatic parenchymal cells by RT-PCR**

| Gene name | Primer Sequences (upstream /downstream) (sense primer/ antisense primer) | Annealing temperature (°C) | Product Size (bp) |
|---|---|---|---|
| GAPDH | AATCCCATCACCATCTTCC (SEQ ID No: 29) | 56 | 382 |
| | CATCACGCCACAGTTTCC (SEQ ID No: 30) | | |
| PEPCK | CTTCGGCAGCGGCTATGGT (SEQ ID NO: 31) | 50 | 383 |
| | TGGCGTTGGGATTGGTGG (SEQ ID No: 32) | | |
| AFP | TTTTGGGACCCGAACTTTCC (SEQ ID No: 33) | 56 | 451 |
| | CTCCTGGTATCCTTTAGCAACT CT (SEQ ID No: 34) | | |
| ALB | GGTGTTGATTGCCTTTGCTC (SEQ ID No: 35) | 56 | 502 |
| | CCCTTCATCCCGAAGTTCAT (SEQ ID No: 36) | | |
| AAT | GGACCTCTGTCTCGTCTTGG (SEQ ID No: 37) | 60 | 183 |
| | GCTCTGATTTGGGGTTGTGT (SEQ ID No: 38) | | |
| TAT | CCCCTGTGGGTCAGTGTT (SEQ ID No:39) | 56 | 345 |
| | GTGCGACATAGGATGCTTTT (SEQ ID No: 40) | | |
| CYP2B6 | AGGGAGATTGAACAGGTGATT (SEQ ID No:41) | 56 | 253 |
| | GATTGAAGGCGTCTGOTTT (SEQ ID No: 42) | | |
| CYP3A7 | CTATGATACTGTGCTACAGT (SEQ ID No: 43) | 50 | 455 |
| | TCAGGCTCCACTTACGGTCT (SEQ ID No: 44) | | |
| KRT8 | GGAGGCATCACCGCAGTAC (SEQ ID No: 45) | 56 | 472 |
| | TCAGCCCTTCCAGGCGAGAC (SEQ ID No: 46) | | |
| KRT18 | GGTCTGGCAGGAATGGGAGG (SEQ ID No: 47) | 56 | 460 |
| | GGCAATCTGGGCTTGTAGGC (SEQ ID No: 48) | | |
| KART7 | TCCGCGAGGTCACCATTAAC (SEQ ID No: 49) | 55 | 218 |
| | GCTGCTCTTGGCCGACTTCT (SEQ ID No: 50) | | |
| OCT3/4 | GAACCGAGTGAGAGGCAACC (SEQ ID No: 51) | 55 | 457 |
| | ATCCCAAAAACCCTOGCACA (SEQ ED No: 52) | | |
| NANOG | TGCCTCACACGGAGACTG (SEQ ID No: 53) | 55 | 353 |
| | GCTATTCTTCGGCCAGTT (SEQ ID No: 54) | | |

In order to further determine whether these hepatocyte-like cells possess hepatic function, a series of function detections were carried out on the cells obtained by differentiation.

It was shown in ELISA detection (the ELISA detection kit was purchased from BETHYL Co. USA) that the albumin secretion amount of the hepatocyte-like cells obtained via differentiation of progenitor cells could reach 439ng/day/million cells, which was close to the albumin secretion amount (439ng/day/ million cells) of the hepatocyte-like cells obtained directly by differentiation of embryonic stem cells (Figure 24).

Glycogen storage status in cells was analyzed by Periodic acid Schiff (PAS) staining. The result showed that the differentiated colonies could be specifically stained in red, which indicated that these hepatocyte-like cells possessed glycogen storage function (Figure 25A).

Moreover, the hepatic parenchyma-like cells obtained by differentiation were tested for their absorption and release status of indocyanine green. It is known that the ability of absorbing and releasing ICG is a specific function of hepatic parenchymal cells, which has been widely used for identifying hepatic parenchymal cells during the differentiation of embryonic stem cells.

The detection method is shown as follow: cells were incubated in a medium containing 1mg/ml indocyanine green (purchased from Sigma-Aldrich Co. USA, I2633-25MG) at 37°C for 15 min; the medium containing indocyanine green was then discharged and the cells were washed with PBS for 3 times; the absorption status of indocyanine green was observed after replacement with fresh medium. Subsequently, the cells were continuously cultured for 6 hours, and the release status of indocyanine green was observed under a microscope after replacement with fresh medium.

The hepatocyte-like cells obtained via differentiation of progenitor cells could absorb the indocyanine green in the medium and showed a green color, then released the indocyanine green absorbed in cells after 6 hours. As a control, the un-differentiated progenitor cells could not absorb indocyanine green (Figure 25B).

A further detection showed that the hepatic parenchyma-like cells obtained via differentiation of progenitor cells could absorb low density lipoprotein (LDL)(Figure 25C).

The detection method is shown as follow: 10µg/ml Dil-Ac-LDL(purchased from Biomedical technologies Co. USA, BT-902) was added to the cells in cultivation; the cells were then cultured at 37°C for 4 hours; the medium containing Dil-Ac-LDL was discharged, and the cells was washed with PBS for 3 times and then observed under a fluorescent microscope after replacement with fresh medium.

The activity of cytochrome p450 in the differentiated cells was analyzed by PROD detection. Without induction by phenobarbital, the cells obtained by differentiation only possessed a weak PROD activity. Phenobarbital induction could improve the PROD activity of the differentiated cells, which demonstrated that differentiated cells indeed possessed the activity of cytochrome p450. As a control, un-differentiated progenitor cells had a very low PROD activity (Figure 25D).

All in all, the above functionality experiments demonstrated that the progenitor cells can differentiate into hepatocyte-like cells that possess certain functions.

Figure 25A was the PAS staining analysis, showing that cytoplasm of the hepatic parenchyma-like cells obtained by differentiation was stained in red, which indicated that these cells stored glycogen. Figure 25B showed that the differentiated cells could absorb ICG (left), and release ICG after 6 hour (middle); whereas the progenitor cells could not absorb ICG (right). Figure 25C showed that the hepatocyte-like cells obtained by differentiation could absorb dil-labeled LDL. Figure 25D showed that without phenobarbital, the differentiated cells only exhibited a weak PROD activity (middle). Through the induction by phenobarbital, PROD activity was enhanced (left), Progenitor cells only exhibited a weak PROD activity (right). (middle), phenobarbital. The scale, 50µm.

### B) Differentiation of hepatic progenitor cells into bile duct-like cells

1) 4.64ml DMEM/F-12 basic culture medium was added to 160µl Matrigel (BD Co. USA, 354230) and mixed well; the mixture was transferred into a culture dish and shaked to cover the entire bottom of the dish; the culture dish was kept at 37°C for 1 hour; the Matrigel solution was discharged before use;
2) the medium was discharged, and the hepatic progenitor cells in good growth state were selected from step A) and washed once with PBS;
3) trypsin-EDTA solution was added to digest the cells at room temperature for 3-5 min; the cells were then observed under a microscope; the cells were ready for use if they contracted into a round shape and were detached from one another;
4) the digestion was terminated by adding DMEM containing 10 (v/v) % fetal bovine serum;
5) the cells were suspended and transferred into a 15ml centrifuge tube, and centrifuged at 1000 rpm for 5 min;
6) the cells were resuspended in a suitable amount of William E medium (Sigma-Aldrich Co. USA, W4128);
7) the hepatic progenitor cells were inoculated onto the culture dish coated with Matrigel of step 1);
8) bile duct differentiation medium, i.e, the medium containing 20mM HEPES, 17mM NaHCO₃, 5mM sodium pyruvate, 0.2mM Asc-2P, 14mM glucose, 1 (v/v) % GlutaMAX-I dipeptide (Invitrogen Co. USA, 35050-061), 0.1µM dexamethasone, 1 (v/v) % msulm-transferrm-sodium selenite mixed supplementary liquid (Gibco Co. USA), 0.05 (w/w) % bovine serum albumin component V, 5.35µg/ml linolenic acid (BD Co. USA, 354227), 20ng/ml EGF, was added;
9) the cells were cultured in an incubator filled with CO₂, with medium refreshed every day.

It has been reported that Matrigel is capable of inducing differentiation of the hepatic progenitor cells obtained directly from separation of embryonic liver into bile duct cells (Tanimizu and Miyajima, J Cell Sci. 2004 Jul 1;117, 3165 - 3174). Immunofluorescent analysis showed that after induction, the cells expressing KRT 19 and KRT7 but not AFP appeared (Figure 26). Figure 26A showed KRT7 positive cells in red; Figure 26B showed KRT19 positive cells in red, The scale was 50µm. This result indicated that the hepatic progenitor cells had the potential to differentiate into bile duct cells.

### C) Differentiation of hepatic progenitor cells into bile duct cells in a three-dimension culture condition

1) the medium was discharged, and the hepatic progenitor cells that grew well were washed once with PBS;
2) trypsin-EDTA solution was added to digest the cells at room temperature for 3-5 min; the cells were then observed under a microscope; the cells were ready for use if they contracted into a round shape and were detached from one another;
3) the digestion was terminated by adding DMEM containing 10 (v/v) % fetal bovine serum; the cells were suspended and transferred into a 15ml centrifuge tube, and centrifuged at 1000 rpm for 5 min;
4) the cells were resuspended in a suitable amount of bile duct differentiation medium;
5) mixed gel was prepared as follow: 1ml of the gel contained 400µl Matrigel, 240µl Type I collagen (R&D Co. USA, 3442-100-01), and 360µl bile duct differentiation medium;
6) the gel was mixed evenly and centrifuged gently in a hand-held centrifuge to avoid generation of air bubbles;
7) the mixed gel was added to the culture dish at 100µl/cm² carefully;
8) the cells were cultured in an incubator at 37°C for 1-2 hours, until solidification of the gel;
9) an equal volume of bile duct differentiation medium was added onto the solidified gel, and the cells were cultured in an incubator at 37°C, with the bile duct differentiation medium on the gel refreshed every day.

After the hepatic progenitor cells differentiated as above for 7 days, the differentiated cells formed a vesicular structure with a central hollow lumen and an outer layer consisting of monolayer cells. It was found in an immunofluorescent detection that two well-known marker proteins KRT7 and KRT19 of bile duct cells were expressed in the monolayer cells of the vesicle, whereas the protein AFP, specific to hepatic lineage, was not expressed.

Furthermore, the subcellular localization of proteins such as β-catenin, E-cadherin, integrin α₆ and F-actin, etc. was detected by an immunofluorescent method, so as to determine whether the differentiated cells have the same polarity of top side to bottom side as that of bile duct cells.

The detection showed that β-catenm was only located at the bottom side of the cells, whereas F-actin was enriched in the inner layer of the vesicle, i.e. the top side. As a result, the differentiated cells that constituted the vesicular structure had an epithelium polarity of top side to bottom side. In addition, E-cadherin and integrinα₆ were also specifically expressed at the bottom side (Figure 27). Figure 27A showed the bile duct-like cells formed a bile duct-like structure; Figure 27B was the result of immunofluorescence, showing that bile duct-like cells expressed KRT19 (in red); Figure 27C was the result of immunofluorescence, showing that bile duct-like cells expressed KRT7 (in red), but did not express AFP (in green); Figure 27D showed the localization of the marker protein β-catenin with an epithelium polarity; Figure 27G showed the localization of the marker protein E-cadherin with an epithelium polarity; Figure 27J showed the localization of Integrin α₆. It was also shown that β-catenin(D), E-cadherin(G) and Integrinα₆(J) were localized at the bottom side of the cells; F-actin (Figure 27E and Figure 27H) was localized at the top side of the cells. Marker KRT19 of bile duct cells was localized at both the top and the bottom sides (Figure 27K). Figures 27F, I, L were merged images. The blue color showed the cellular nuclei labeled with DAPI. The scale was 50µm.

In order to detect whether the bile duct-like cells obtained by differentiation have the same transportation and secretion function as normal bile duct cells, the function of the key protein MDR involved in the transportation and secretion in bile duct was analyzed. MDR is a ATP-dependent transmembrane transportation pump, which has been reported to participate the secretion of the cationic substances in bile (Gigliozzi et al., Gastroenterology. 2000 Oct; 119,1113 - 1122). The vesicles obtained by differentiation were co-incubated with a fluorescent dye rhodamine 123 (Sigma-Aldrich Co. USA, 83702-10MG). The fluorescent intensity in the hollow lumen of the vesicle was much higher than that in the surrounding cells. After the treatment with 10mM MDR protein inhibitor, i.e. Verapamil(Sigma-Aldrich Co. USA, V106-SMG), rhodamine 123 was limited within the peripheral cells of the vesicle and lost the ability to be transported into the hollow lumen of the vesicle (Fig. 28), which demonstrated the transportation of rhodamine 123 is indeed dependent on the functional MDR protein located at the top side of cells. The above results indicated that these cells that were obtained by differentiation of hepatic progenitor cells had a high similarity with bile duct cells.

Hepatic progenitor cells can also be obtained by differentiation of induced pluripotent stem (iPS) cell lines hAFF-4U-M-iPS-1 and hAFF-4U-M-iPS-3(Yang Zhao, Two supporting factors greatly improve the efficiency of human iPSC generation. Cell Stem Cell, 2008;3:475-479.) (Peking University) in the same way. These hepatic progenitor cells also have clone morphology and a long-term proliferation ability; as well as express AFP, KRT19(Figure 30) and KRT7, and the presumed hepatic progenitor cell markers EpCAM and CD133.

## Claims

1. A method for inducing the differentiation of embryonic stem cells (ESC) or induced pluripotent stem cells (iPS cells) into hepatocytes, comprising the following steps:
1) culturing said embryonic stem cells (ESC) or induced pluripotent stem cells (iPS cells) in a basic cell culture medium containing activin A;
2) transferring the cells obtained in step 1) into a basic cell culture medium containing insulin-transferrin-selenium salt (preferably sodium selenite) and activin A for cultivation;
3) culturing the cells obtained in step 2) in a hepatocyte culture medium (HCM) containing fibroblast growth factor (FGF) and bone morphogenetic protein (BMP), so as to generate hepatic progenitor cells; and
4) promoting the maturation of said hepatic progenitor cells obtained in step 3), so as to generate hepatocytes,
wherein preferably said embryonic stem cells (ESC) or induced pluripotent stem cells (iPS cells) are mammal cells, more preferably mouse or human cells, and most preferably human cells, wherein in the case that said cells are human cells, preferably said activin A is human activin A, said fibroblast growth factor is human fibroblast growth factor, and said bone morphogenetic protein is human bone morphogenetic protein.

2. The method according to claim 1, wherein said basic cell culture medium is selected from the group consisting of MEM, DMEM, BME, DMEM / F12, RPMI1640 and Fischer's.

3. The method according to claim 1 or 2, wherein the concentration of said activin A in said basic cell culture medium is 10∼500 ng/ml.

4. The method according to claim 3, wherein said insulin-transferrin-selenium salt is added as a mixed supplementary liquid, and the volume ratio of said insulin-transferrin-selenium salt to said basic cell culture medium is 0.01∼20%.

5. The method according to claim 4, wherein said fibroblast growth factor is acidic fibroblast growth factor, fibroblast growth factor 2 or fibroblast growth factor 4; said bone morphogenetic protein is bone morphogenetic protein 2 or bone morphogenetic protein 4.

6. The method according to claim 5, wherein the amount of said fibroblast growth factor (FGF) is 5∼100 ng/ml said hepatocyte culture medium; and the amount of said bone morphogenetic protein (BMP) is 5∼100 ng/ml said hepatocyte culture medium.

7. The method according to claim 6, wherein promotion of maturation of said hepatocytes is carried out by culturing said hepatocytes in a hepatocyte culture medium containing a hepatocyte growth factor ( preferably human hepatocyte growth factor) and a keratinocyte growth factor ( preferably human keratinocyte growth factor) so as to obtain proliferated hepatic progenitor cells; transferring the hepatic progenitor cells into a hepatocyte culture medium containing oncostatin M and dexamethasone for cultivation, then transferring the cells into a differentiation medium V for cultivation and obtaining mature hepatocytes; wherein said differentiation medium V is a basic culture medium containing (0.1-10)ml/100ml N2, (0.1-20 )ml/100ml B27, 0.5-2mM glutamine, (0.1-10)ml/100ml nonessential amino acid, 0.05-0.2 mM β-mercaptoethanol, 1-100ng/ml oncostatin M(OSM) and 0.05-1µM dexamethasone (Dex), pH 7.2-7.6.

8. The method according to claim 7, wherein the amount of said hepatocyte growth factor is 5∼100 ng/ml said hepatocyte culture medium; the amount of said keratinocyte growth factor is 5∼100 ng/ml said hepatocyte culture medium, the amount of said oncostatin M is 1∼100 ng/ml said hepatocyte culture medium; the concentration of said dexamethasone in said hepatocyte culture medium is 0.05∼1 µM.

9. Hepatocytes obtained by the method according to any of claims 1-8, wherein preferably said hepatocytes express marker molecules AFP, Alb, CK8, CK18, CK19, HNF4α, and/or GAPDH of hepatocytes, more preferably said hepatocytes have glycogen synthesis and storage function, urea synthesis function, leukocyte secretion function and/or P450 enzyme activity in response to drug induction.

10. Use of the hepatocytes obtained by the method according to any of claims 1-8 in preparation of artificial livers, test of drug toxicity or drug screening.

11. A method for inducing the differentiation of embryonic stem cells (ESC) or induced pluripotent stem cells (iPS cells) into hepatic endoderm cells, comprising the following steps:
1) culturing said embryonic stem cells (ESC) or induced pluripotent stem cells (iPS) in a basic cell culture medium containing activin A;
2) transferring the cells obtained in step 1) into a basic cell culture medium containing insulin-transferrin-selenium salt (preferably sodium selenite ) and activin A for cultivation; and
3) culturing the cells obtained in step 2) in a hepatic endoderm cell inducing medium containing fibroblast growth factor (FGF) and bone morphogenetic protein (BMP), so as to generate hepatic endoderm cells,
wherein preferably said embryonic stem cells (ESC) or induced pluripotent stem cells (iPS cells) are mammal cells, more preferably mouse or human cells, and most preferably human cells, wherein in the case that said cells are human cells, preferably said activin A is human activin A, said fibroblast growth factor is human fibroblast growth factor, and said bone morphogenetic protein is human bone morphogenetic protein.

12. The method according to claim 11, wherein said basic cell culture medium used in steps 1) and 2) further contains bovine serum albumin component V, wherein preferably said fibroblast growth factor is acidic fibroblast growth factor, fibroblast growth factor 2 or fibroblast growth factor 4; and said bone morphogenetic protein is bone morphogenetic protein 2 or bone morphogenetic protein 4.

13. The method according to claim 11 or 12, wherein in step 2), the cells obtained in step 1) are first transferred into a basic cell culture medium containing insulin-transferrin-selenium salt at a first concentration and activin A so as to culture the cells, then the resultant cells are cultured in a basic cell culture medium containing insulin-transferrin-selenium salt at a second concentration and activin A, said second concentration is equal or higher than said first concentration.

14. The method according to claim 13, wherein the medium used in step 1) is a basic cell culture medium containing 0.02%-1% w/w of bovine serum albumin component V and 50-200 ng/ml human activin A; the medium used in step 2) is a basic cell culture medium containing 0.02%-1% w/w of bovine serum albumin component V, 0.05%-0.5% v/v of insulin-transferrin-sodium selenite mixed supplementary liquid and 50-200ng/ml human activin A, and a basic cell culture medium containing 0.02%-1% w/w of bovine serum albumin component V, 0.5%-2% v/v of insulin-transfenin-sodium selenite mixed supplementary liquid and 50-200ng/ml human activin A, respectively; said hepatic endoderm cell inducing medium is a hepatocyte culture medium containing 20-60ng/ml human fibroblast growth factor -4 and 10-30ng/ml human bone morphogenetic protein -2.

15. The method according to any of claims 11-14, wherein said basic cell culture medium is selected from the group consisting of MEM, DMEM, BME, DMEM / F12, RPMI1640 and Fischer's.

16. The method according to claim 11, further comprising a step of sorting the cells expressing the surface protein N-cadherin by using a flow cytometer after step 3).

17. The method according to claim 11, wherein the cells are cultured for 24 hours, 48 hours and 5 days in steps 1), 2) and 3), respectively.

18. The method according to claim 11, wherein said embryonic stem cell (ESC) is human embryonic stem cell, said human embryonic stem cell is commercially available human embryonic stem cell line; preferably is one of the following cell lines: BG01, BG02, BG03, BG04, SA01, SA02, SA03, ES01, ES02, ES03, ES04, ES05, ES06, TE03, TE32, TE33, TE04, TE06, TE62, TE07, TE72, UC01, UC06, WA01, WA07, WA09, WA13 and WA14; wherein the accession numbers are NIH numbers.

19. Hepatic endoderm cells obtained by the differentiation of human embryonic stem cells or human induced pluripotent stem cells by the method according to any of claims 11-18, wherein preferably the hepatic endoderm cells express at least 3 types of marker protein of hepatic endoderm cells, i.e. α-fetoprotein, hepatocyte nuclearfactor 4A and N-cadherin.

20. The hepatic endoderm cells according to claim 19, wherein said hepatic endoderm cells express α-fetoprotein, albumin, hepatocyte nuclearfactor 4A, hepatocyte nuclear factor 3B and N-cadherin.

21. Use of the hepatic endoderm cells according to claim 19 or 20 in preparation of hepatocyte-like cells or cholangiocyte-like cells.

22. A method for inducing the differentiation of embryonic stem cells (ESC) or induced pluripotent stem cells (iPS cells) into hepatic progenitor cells, comprising the following steps:
1) culturing said embryonic stem cells (ESC) or induced pluripotent stem cells (iPS cells) in a basic cell culture medium containing activin A;
2) transferring the cells obtained in step 1) into a basic cell culture medium containing insulin-transferrin-selenium salt (preferably sodium selenite ) and activin A for cultivation;
3) culturing the cells obtained in step 2) in a hepatic endoderm cell inducing medium containing fibroblast growth factor (FGF) and bone morphogenetic protein (BMP), so as to generate hepatic endoderm cells; and
4) culture the hepatic endoderm cells obtained in step 3) with a hepatic progenitor cell culture medium on STO cell feeder layer,
wherein preferably said embryonic stem cells (ESC) or induced pluripotent stem cells (iPS cells) are mammal cells, more preferably mouse or human cells, and most preferably human cells, wherein in the case that said cells are human cells, preferably said activin A is human activin A, said fibroblast growth factor is human fibroblast growth factor, and said bone morphogenetic protein is human bone morphogenetic protein.

23. The method according to claim 22, wherein said basic cell culture medium used in steps 1) and 2) further contains bovine serum albumin component V, wherein preferably said fibroblast growth factor is acidic fibroblast growth factor, fibroblast growth factor 2 or fibroblast growth factor 4; said bone morphogenetic protein is bone morphogenetic protein 2 or bone morphogenetic protein 4.

24. The method according to claim 22 or 23, wherein in step 2), the cells obtained in step 1) are first transferred into a basic cell culture medium containing insulin-transferrin-selenium salt at a first concentration and activin A so as to culture the cells, then the resultant cells are cultured in a basic cell culture medium containing insulin-transferrin-selenium salt at a second concentration and activin A, said second concentration is higher than said first concentration.

25. The method according to claim 24, wherein the medium used in step 1) is a basic cell culture medium containing 0.02%-1 % w/w of bovine serum albumin component V and 50-200 ng/ml human activin A; the medium used in step 2) is a basic cell culture medium containing 0.02%-1% w/w of bovine serum albumin component V, 0.05%-0.5% v/v of insulin-transferrin-sodium selenite mixed supplementary liquid and 50-200ng/ml human activin A, and a basic cell culture medium containing 0.02%-1% w/w of bovine serum albumin component V, 0.5%-2% v/v of insulin-transferrin-sodium selenite mixed supplementary liquid and 50-200ng/ml human activin A, respectively; said hepatic endoderm cell inducing medium is a hepatocyte culture medium containing 20-60ng/ml human fibroblast growth factor -4 and 10-30ng/ml human bone morphogenetic protein -2, said hepatic progenitor cell culture medium is a basic cell culture medium containing 5-25 mM HEPES, 0.5%-2% v/v of insulin-transferrin-sodium selenite mixed supplementary liquid, 0.02%-1% w/w of bovine serum albumin component V, 2-20 mM niacinamide, 0.2-2mM diphosphorylated ascorbic acid, 0.02-0.2µM dexamethasone, and 5-40ng/ml EGF.

26. The method according to any of claims 22-25, wherein said basic cell culture medium is selected from the group consisting of MEM, DMEM, BME, DMEM / F12, RPMI1640 and Fischer's.

27. The method according to claim 22, further comprising a step of sorting the cells expressing the surface protein N-cadherin by using a flow cytometer after step 3).

28. The method according to claim 22, wherein the cells are cultured for 24 hours, 48 hours and 5 days in steps 1), 2) and 3), respectively.

29. The method according to any of claims 22-28, further comprising a passage step of the hepatic progenitor cells; the method for the passage of the hepatic progenitor cells comprises the steps of digesting said hepatic progenitor cells with trypsin-EDTA solution, and culturing the resultant cells on a hepatic progenitor cell culture medium with STO cells as the feeder layer.

30. The method according to claim 22, wherein said embryonic stem cell (ESC) is human embryonic stem cell, said human embryonic stem cell is commercially available human embryonic stem cell line; preferably is one of the following cell lines: BG01, BG02, BG03, BG04, SA01, SA02, SA03, ES01, ES02, ES03, ES04, ES05, ES06, TE03, TE32, TE33, TE04, TE06, TE62, TE07, TE72, UC01, UC06, WA01,WA07, WA09, WA13 and WA14; wherein the accession numbers are NIH numbers.

31. Hepatic progenitor cells obtained by the differentiation of human embryonic stem cells or human induced pluripotent stem cells by the method according to any of claims 22-30, wherein preferably the hepatic progenitor cells are hepatic progenitor cells expressing α-fetoprotein, keratin 19 and keratin 7, and possess a proliferation ability and a dual-directional differentiation potential towards hepatocyte-like cells and cholangiocyte-like cells.

32. Use of the hepatic progenitor cells according to claim 31 in preparation of hepatocyte-like cells or cholangiocyte-like cells.
